# EUROPEAN PATENT APPLICATION

(11) **EP 3 915 548 A1**
(43) Date of publication of application: **01.12.2021**
(21) Application number: 21175831.3
(22) Date of filing: 25.05.2021
(51) Int. Cl.: A61K 9/20, A61K 31/706, A61P 31/00

(54) **TRANSMUCOSAL PHARMACEUTICAL COMPOSITIONS OF ANTIVIRAL DRUGS**

(30) Priority: 29.05.2020 IN 202011022634; 15.07.2020 WO PCT/IN2020/050609; 03.09.2020 IN 202011038103
(71) Applicant: Jubilant Generics Limited, 201 301 Uttar Pradesh (IN)
(72) Inventor: Nandi, Indranil, Yardley PA, 19067 (US); Kumar, Dinesh, Noida, UP (IN); Jain, Anil, 201 301 Noida, UP (IN); Jaiswal, Nilesh, 201 301 Noida, UP (IN); Mukherjee, Tusharmouli, Yardley PA, 19067 (US); Soni, Pankaj, 201 301 Noida, UP (IN); Mistry, Gaurav Navinbhai, 201 301 Noida, UP (IN)
(74) Representative: Herrero & Asociados, S.L.

(57) **Abstract**

The present invention relates to the transmucosal dosage forms like sublingual pharmaceutical compositions comprising antiviral molecules like favipiravir, remdesivir, baloxavir marboxil, molnupiravir, besifovir, raltegravir, GS-441524, ravidasvir, and other antiviral drugs. The present invention also relates to methods for preparing these transmucosal pharmaceutical compositions. Compositions prepared as per the present invention are able to increase bioavailability by avoiding first-pass metabolism. The compositions prepared as per the present invention exhibit desired pharmaceutical technical attributes such as pH, assay, related substance, disintegration, and dissolution. The compositions prepared as per the present invention are useful in the treatment of viral infections including coronavirus infection (COVID-19).

## Description

### FIELD OF THE INVENTION

The present invention generally relates to the field of pharmaceutical sciences. Specifically, the present invention relates to transmucosal pharmaceutical compositions like sublingual dosage forms of one or more antiviral drugs or their pharmaceutically acceptable salts or solvates thereof. In particular, but without restriction to the particular embodiments hereinafter described in accordance with the best mode of practice, the present invention provides sublingual pharmaceutical compositions comprising one or more antiviral drugs and processes for preparing these compositions. The present invention also provides sublingual pharmaceutical compositions and processes for preparing these compositions to increase bioavailability by avoiding first-pass metabolism and reduce the variation in bioavailability. The compositions as per the present invention are useful for treating and/or preventing viral infections including coronavirus infections (SARS CoV-2, also called COVID-19).

### BACKGROUND OF THE INVENTION

The virus infections are among the major causes of human morbidity and severely threatens global public health, affecting social stability and economic development. Viruses are non-specific cell structures and rely entirely on the energy and metabolic systems of host cells to acquire the substances and energy required for life activities.

The coronavirus is a single-stranded positive RNA virus that adversely impacts the upper respiratory tract in patients with immune deficiencies like pneumonia, exacerbation of asthma, and neurological symptoms. A recently identified highly contagious novel coronavirus (COVID-19) is known to cause severe and life-threatening acute respiratory syndrome (SARS). Coronavirus (COVID-19) pandemic has adversely impacted millions of lives globally and also adversely influenced the global economy due to lockdown in various countries.

Due to very high disease-related morbidity and mortality, various treatment options are being extensively studied and researched in recent times for the treatment of coronavirus infection (including COVID-19 disease). Various drugs, biologics, and vaccines are under development as a treatment option for this life-threatening disease. Regulatory authorities have also become more surveilling concerning the development and approval of breakthrough treatment options for coronavirus infection. Among antiviral drugs, molecules like favipiravir, remdesivir, molnupiravir, and GS-441524 are being considered as a treatment option for COVID-19 infection.

Innovator Gilead's molecule remdesivir at present is an important molecule for the treatment of COVID-19 infection. Remdesivir is known to exhibit antiviral properties. Remdesivir is a single stereoisomer mono-phosphoramidate prodrug of a nucleoside analog, that is developed for the treatment of coronavirus disease. Remdesivir is chemically known as 2-Ethylbutyl (2S)-2-{[(S)-{[(2R,3S,4R,5R)-5-(4-aminopyrrolo[2,1-f][1,2,4]triazm-7-yl)-5-cyano-3,4-dihydroxytetra hydrofuran-2-yl] methoxy} (phenoxy)phosphoryl] amino} propanoate. Remdesivir is a white to off-white or yellow non-hygroscopic solid. It is practically insoluble in water and is represented by the following formula as:

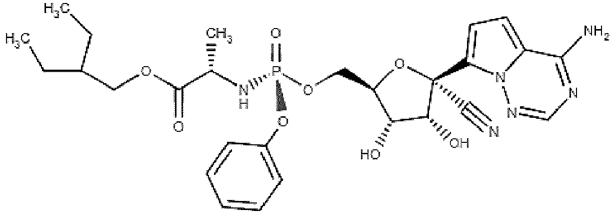

Remdesivir is a prodrug that metabolizes into its active form GS-441524. The active metabolite GS-441524 is an adenosine nucleotide analog that interferes with the action of viral RNA polymerase and evades proofreading by viral exoribonuclease, causing a decrease in viral RNA production. Literatures also disclose that GS-441524, which is a main plasma metabolite of remdesivir shows antiviral properties in COVID-19 treatment.

Remdesivir is presently approved in many countries in the form of an injectable dosage form in the strength of 100 mg by Gilead Sciences for the treatment of infection caused by coronavirus disease 2019 (COVID-19). Remdesivir is reported to be poorly water-soluble, which leads to poor dissolution and poor bioavailability. Moreover, remdesivir is not the first choice for oral delivery as its shows poor hepatic stability, which results in its complete first-pass clearance. These characteristics of remdesivir pose technical challenges to formulation scientists in the development of a suitable oral formulation with desired technical attributes.

PCT Patent Publication No. WO2016/069826 assigned to Gilead Sciences discloses remdesivir as a compound per se and its manufacturing process. This patent publication also discloses the use of remdesivir in the treatment of Filoviridae infection.

US Patent Publication No. US2019/0255085 assigned to Gilead Sciences discloses the use of remdesivir in the treatment of Coronaviridae infection in a human.

US Patent Publication No. US2018/346504 assigned to Gilead Sciences discloses crystalline forms of remdesivir. This patent publication further discloses crystalline form I to form IV, mixtures of crystalline forms and maleate form I of remdesivir.

US Patent Publication No. US2019/083525 assigned to Gilead Sciences discloses remdesivir injectable dosage form. However, the said patent publication fails to disclose any solid oral composition of remdesivir.

These publications fail to disclose how to overcome the technical challenges associated with remdesivir such as poor solubility or the importance of pH on its solubility to be considered for designing oral dosage forms. Further, these publications also fail to disclose transmucosal dosage forms such as sublingual and successful dosage form development with desired formulation technical attributes.

Oral dosage forms always remain the first choice of route for drug administration among all age groups of patients and healthcare professionals. However, the development of oral dosage forms for most antiviral drugs is challenging due to their undesirable physicochemical properties such as poor solubility and complete first-pass metabolism. Further, dysphagia due to large tablet size is another common problem associated with oral administration, especially for geriatric, paediatric, and hospitalized patients. These patients may have difficulty in swallowing due to severe viral infection and consequently these patients may not benefit from these antiviral drugs.

For example, the development of oral dosage forms of antiviral drugs such as remdesivir is not feasible and is challenging due to its adverse physicochemical properties such as poor solubility and complete first-pass metabolism. Due to these technical difficulties, drugs like remdesivir are approved only for intravenous injection. The approved injection product is indicated for patients who require hospitalization for the treatment of coronavirus disease (COVID-19). Thus, the injection would be required to be administered in a hospital or in a healthcare setting capable of providing acute care comparable to inpatient hospital care which requires huge and advanced medical infrastructure and set-ups, which is highly challenging for least developed countries and countries having a significant geographical area and large population. Further, the dose of remdesivir injection is 200 mg on day 1, followed by once-daily maintenance doses of 100 mg from day 2. Thus, administration of remdesivir injection in hospitalized patients is again a big challenge and accordingly requires trained and highly skilled medical professionals. Further, the said injection is administered only via intravenous infusion within 30 to 120 minutes. The treatment duration is usually for 5 days and may be extended up to 5 additional days (10 days total) if clinical improvement is not observed. Thus, the whole process may be undesirable to patients due to low patient compliance. Further, the injection dosage form requires special storage conditions, such as the diluted injection solution in the infusion bags are required to be stored up to 24 hours at room temperature before administration or 48 hours at refrigerated temperature (2°C to 8°C) which not only requires specialized infrastructure but also increases the product cost.

Many alternative routes of drug administration are available to bypass the first-pass metabolism such as ophthalmic, injectable, nasal, transdermal, topical, buccal, and sublingual routes. In the sublingual administration route, the patient needs to keep the pharmaceutical composition under the tongue while the drug diffuses into the mouth, through the mucosa lining, and from there into the bloodstream. As an alternative to the solid oral dosage form or gastrointestinal delivery of the drug, sublingual administration might conceivably increase the bioavailability of remdesivir by avoiding first-pass hepatic metabolism.

Rapid absorption of the drug by the oral mucosa and low drug loading ability are some of the key requirements for the formulation design of sublingual dosage forms. In addition, antiviral drugs have an unpleasant taste, such as a bitter taste, which may cause numbness and contraction, and the oral or sublingual administration puts a burden on a patient and lowers compliance. The unpleasant taste may stimulate saliva flow which leads to increased swallowing of the drug. This undesirable taste factor negatively influences patient compliance, especially with paediatric and geriatric patients. In view of the foregoing, there is an unmet need for improvement in the methods of administering antiviral drugs to increase the bioavailability of the drug, reduce variability in dosing, and improved patient compliance.

The present inventors have successfully designed and developed transmucosal pharmaceutical compositions of antiviral drugs. The proposed compositions as per the present invention have desirable formulation technical attributes and comply with the demanding requirements and regulations of health and medicine regulatory agencies across the world. Further, the process employed in the manufacture of the dosage form is consistent, cost-effective, and therefore feasible for industrial production. Further, sublingual delivery is likely to provide more consistent absorption than oral delivery. The prepared dosage forms are suitable for the treatment of viral infections caused by members of the filoviridae, flaviviridae, paramyxoviridae, orthomyxoviridae, coronaviridae (including COVID-19 infection), arenaviridae, and/or adenoviridae families. Antiviral drugs such as remdesivir, favipiravir, baloxavir marboxil, besifovir, raltegravir, molnupiravir, GS-441524, and ravidasvir are included as being suitable for inclusion in the transmucosal pharmaceutical compositions described herein.

### SUMMARY OF THE INVENTION

The present invention relates to transmucosal dosage forms like sublingual pharmaceutical compositions of antiviral drugs and one or more pharmaceutically acceptable excipients and processes for preparing such compositions.

The present invention also relates to transmucosal dosage forms like sublingual pharmaceutical composition comprising an antiviral drug such as remdesivir, favipiravir, baloxavir marboxil, besifovir, raltegravir, molnupiravir, GS-441524, and ravidasvir, and one or more pharmaceutically acceptable excipients selected from the group comprising diluent, binder, disintegrant, lubricant, glidant, surfactant, pH regulating agent, effervescent agent, salivating agent, solubilizing agent, stabilizer, sweetener, taste masking agent, preservative, flavoring agent, coloring agent, film-forming agent, and solvent or mixtures thereof.

The present invention also relates to a sublingual pharmaceutical composition comprising remdesivir or its pharmaceutically acceptable salts or solvates thereof and one or more pharmaceutically acceptable excipients and process for preparing such compositions.

The present invention also relates to sublingual pharmaceutical compositions comprising remdesivir or its pharmaceutically acceptable salts or solvates thereof and one or more pharmaceutically acceptable excipients selected from the group comprising diluent, binder, disintegrant, lubricant, glidant, surfactant, pH regulating agent, effervescent agent, salivating agent, solubilizing agent, stabilizer, sweetener, taste masking agent, preservative, flavoring agent, coloring agent, film-forming agent, and solvent or mixtures thereof.

The compositions as per the present inventions exhibit desired pharmaceutical technical attributes such as one or more of wetting time, pH, disintegration, dissolution, thickness, diameter, hardness, friability, compressibility index, assay, related substance, content uniformity, stability, therapeutic equivalence, patient compliance, and commercial viability and other requirements also.

The present invention also relates to the use of sublingual pharmaceutical compositions in the manufacture of a medicament for treating viral infections caused by members of the filoviridae, flaviviridae, paramyxoviridae, orthomyxoviridae, coronaviridae (including COVID-19 infection), arenaviridae, and/or adenoviridae families.

### DETAILED DESCRIPTION OF THE INVENTION

The present invention can be more readily understood by reading the following detailed description of the invention and study of the included examples.

As used herein, the term "composition", or "dosage form" or "drug delivery system", as in pharmaceutical composition, is intended to encompass a drug product comprising one or more antiviral drugs and other inert ingredient(s) (e.g., pharmaceutically acceptable excipients). Such pharmaceutical compositions are synonymous with "formulation" and "dosage form" of the present invention. It can be administered in any pharmaceutical dosage form that can be held in the mouth for a suitable period of time and permits diffusion or erosion of the drug into the mouth cavity where the drug can be absorbed through the mucosa lining of the mouth. Such dosage forms include transmucosal dosage forms, tablets, lozenges, sublingual tablets, sublingual capsule, sublingual film, sublingual aerosol, sublingual solution, sublingual spray, buccal tablets, buccal capsules, mucoadhesive tablets, bioadhesive tablets, troches, pastilles, pills, viscous liquids, pastes, drops, gels, patches and the like. In an embodiment, the composition is a sublingual spray.

The term "transmucosal dosage form" as used herein refers to a dosage form wherein drug delivery occurs via the transmucosal route such as the sublingual route and not via swallowing followed by gastro-intestinal (GI) absorption.

"Sublingual administration" may be defined herein as the therapeutic administration of a pharmaceutical composition under the tongue. Preferably, the pharmaceutical composition refers to sublingual tablets.

"Buccal compositions" or "Buccal administration" as per the present invention are defined herein as the therapeutic administration of a pharmaceutical composition when placed between the lip, cheeks, and the gum and allowed to dissolve, or otherwise release its active component. The tablet dosage form as per the present invention can be used for both sublingual and/or buccal administration or either of them.

In another embodiment, the composition can be film-coated or uncoated. Preferably, the composition is uncoated. In another embodiment, the composition can be a scored or unscored tablet. Preferably, the tablet is unscored. Preferably, the pharmaceutical compositions as per the present invention are intended for immediate or quick release. Preferably, the sublingual tablet compositions as per the present invention dissolve or disperse in the mouth of the subject in from about 1 second to 360 seconds or 240 seconds. In a further embodiment, the composition as per the present invention is not an injectable composition and/or is not intended for parenteral administration. In a further embodiment, the composition as per the present invention is not an orally disintegrating formulation, wherein the drug is absorbed through GI (gastrointestinal) tract. In a further embodiment, the composition as per the present invention provides sublingual dosage form to dissolve such that the drug diffuses into the mouth for absorption through the mucosa lining of the mouth into the bloodstream to avoid the first-pass metabolism of the drug.

As used herein, the term "antiviral drugs or agents" refers to compounds that are effective in interfering with the virus using any of the mechanisms such as virus penetration of eukaryotic cells, virus replication in eukaryotic cells, virus assembly, virus release from infected eukaryotic cells or inhibiting a virus titer increase or in unspecifically reducing a virus titer level in a eukaryotic or mammalian host system.

"Antiviral" is used in the broad sense to include not only per se compound (free base) but also its pharmaceutically acceptable salts, solvates, esters, hydrates, isomers, enantiomers, stereoisomers, diastereoisomers, derivatives, metabolites, polymorphs, and prodrugs thereof. Polymorph may refer to various crystalline and amorphous forms, which can be characterized by methods such as melting point, X-ray diffraction pattern, Raman spectra, IR spectra, or any other method known in the art.

The following antiviral drugs or their combinations are covered under the ambit of the present invention, including but not limited to, abacavir, abacavir sulfate/lamivudine/zidovudine, abacavir sulfate/dolutegravir sodium/lamivudine, ABI-H0731, AB-423, ABX464, AL-034, acyclovir, adefovir, amantadine, amprenavir, ampligen, arbidol, APG-1387, asunaprevir, atazanavir, AT-527, atazanavir sulfate/cobicstat, baloxavir marboxil, bictegravir, bictegravir/emtricitabine/tenofovir, BIT-225, berdazimer sodium, besifovir, boceprevir, brincidofovir, BLD-2660, cabotegravir, cantharidin, cidofovir, celgosivir, cobicstat, cobicstat/darunavir, cobicstat/elvitegravir /emtricitabine/tenofovir, cyclopropavir, darunavir, daclatasvir, daclatasvir/asunaprevir, daclatasvir/sofosbuvir, dasabuvir, dasabuvir sodium/ombitasvir/paritaprevir/ritonavir, delavirdine, didanosine, docosanol, dolutegravir, dolutegravir/rilpivirine, dolutegravir/lamivudine, doravirine, doravirine/lamivudine/tenofovir disoproxil fumarate, edoxudine, EDP-938, EIDD-2801, efavirenz, efavirenz/emtricitabin/ tenofovir disoproxil fumarate, elbasvir/grazoprevir, elvitegravir, elvitegravir/ritonavir, elsulfavirine, emtricitabine, emtricitabine/tenofovir disoproxil fumarate, emtricitabine/rilpivirine hydrochloride/tenofovir disoproxil, enfuvirtide, entecavir, ENU200, etravirine, EYP-001, faldaprevir, famciclovir, favipiravir, fomivirsen, fosamprenavir, foscarnet, fostemsavir, ganciclovir, galidesivir, glecaprevir, glecaprevir/pibrentasvir, grazoprevir, GS-5801, GS-441524, ibacitabine, imunovir, inarigivir, idoxuridine, imiquimod, inarigavir, indinavir, inosine, INO-9112, interferon type III, interferon type II, interferon type I, islatravir, ISR-50, JNJ-379, JNJ-5806, JNJ-678, lamivudine, laninamivir, lamivudine/raltegravir, ledipasvir, ledipasvir/sofosbuvir, lenacapavir, letermovir, levovir, lomibuvir, lonafarnib, lopinavir, lopinavir/ritonavir, loviride, lumicitabine, maribavir, maraviroc, moroxydine, morphothiadine, methisazone, mericitabine, MK-4250, modipafant, molnupiravir, nitazoxanide, nelfinavir, nevirapine, nexavir, niclosamide, ombitasvir, ombitasvir/paritaprevir/ritonavir, ombitasvir/dasabuvir/ paritaprevir/ritonavir, oseltamivir, paritaprevir, PC-786, peginterferon alfa- 2a, penciclovir, peramivir, pleconaril, pimodivir, podophyllotoxin, podofilox, presatovir, pritelivir, QL-007, raltegravir, ranpirnase, ravidasvir, remdesivir, ribavirin, rimantadine, rilpivirine, ritonavir, RG7863, RG-7907, RG-6004, RG-7854, RV-521, pyramidine, saquinavir, selgantolimod, sinecatechins, simeprevir, sofosbovir, sofosbuvir / velpatasvir, sofosbuvir / velpatasvir / voxilaprevir, sofosbuvir/ribavirin, sofosbuvir/simeprevir, sorivudine, stavudine, tecovirimat, telaprevir, telbivudine, tenofovir, tenofovir disoproxil, tenofovir alafenamide fumarate, tenofovir disoproxil orotate, tenofovir disoproxil aspartate, tenofovir exalidex, teslexivir, tipranavir, TMB-607, trifluridine, trizivir, tromantadine, vaniprevir, vaniprevir/ribavirin, valacyclovir, valganciclovir, vesatolimod, vicriviroc, vidarabine, viramidine, zalcitabine, zanamivir, zidovudine, ziresovir, zotatifin, 2.10 genekam-T-010, 2.8 genekam-T-008, 2.9 genekam-T-009, and/or any combinations thereof.

The pharmaceutical acceptable salts of antiviral drugs as per the present invention include, but are not limited to, hydrochloric acid, citric acid, hydrobromic acid, fumaric acid, acetic acid, ascorbic acid, capric acid, alginic acid, cinnamic acid, adipic acid, boric acid, caprylic acid, gentisic acid, succinic acid, amino acids salts, tannic acid, oxalic acid, phosphoric acid, salicylic acid, caproic acid, benzoic acid, cyclamic acid, benzenesulfonic acid, stearic acid, nicotinic acid, nitric acid, lactic acid, mandelic acid, palmitic acid, glycolic acid, pamoic acid, L-tartaric acid, lactobionic acid, saccharic acid, 4-amino-salicylic acid, glucoheptonic acid, formic acid, ethanesulfonic acid, hippuric acid, galactaric acid, orotic acid, thiocyanic acid, camphorsulfonic acid, p-toluenesulfonic acid, cyclohexanesulfamic acid, dodecylsulfuric acid, pyroglutamic acid, malonic acid, sulfuric acid, camphoric acid, oleic acid, hydroiodic acid, L-glutamic acid, malic acid, maleic acid, methanesulfonic acid, valeric acid, undecylenic acid, perchloric acid, D-gluconic acid and sebacic acid and mixtures thereof.

As used herein, the term "remdesivir" is used in the broad sense to include not only "remdesivir" per se (free base) but also its pharmaceutically acceptable salts, solvates, esters, hydrates, isomers, enantiomers, stereoisomers, diastereoisomers, derivatives, metabolites, polymorphs, and prodrugs thereof. Remdesivir and its nucleoside analog/metabolite GS-441524 are covered under the scope of the present invention.

The pharmaceutical compositions of the present invention comprise about 0.1 mg to about 2400 mg of one or more antiviral drugs, preferably about 0.1 mg to about 2000 mg, preferably about 0.1 mg to about 1000 mg, most preferably about 0.1 mg to about 500 mg of the antiviral drug as described herein. Preferably, compositions of the present invention comprise about 0.1 mg to about 200 mg of one or more antiviral drugs. More preferably, compositions of the present invention comprise about 0.1 mg to about 50 mg of one or more antiviral drugs.

The pharmaceutical compositions of the present invention comprise about 0.1 mg to about 500 mg of remdesivir, preferably about 0.1 mg to about 450 mg, preferably about 0.1 mg to about 400 mg, preferably about 0.1 mg to about 350 mg, preferably about 0.1 mg to about 200 mg, preferably about 0.1 mg to about 100 mg and more preferably about 0.1 mg to about 50 mg remdesivir as described herein. In a further embodiment, compositions of the present invention comprise about 0.1 mg to about 20 mg of remdesivir. In a preferred embodiment, compositions of the present invention comprise about 20 mg of remdesivir. The dose may be administered one or more times a day (such as from one to ten times per day).

q.d. (quaque die) means once a day. b.i.d. (bis in die) means two times a day. t.i.d. (ter in die) means three times a day. q.i.d. (quater in die) means four times a day.

The term "excipient" means a pharmacologically inactive component such as diluents, binders, disintegrants, lubricants, glidants, surfactants, wetting agents, pH regulating agents, buffers, taste masking agents/taste masker, water-soluble and/or water-dispersible carrier materials, effervescent agents, salivating agents, antioxidants, permeation/penetration enhancers, solubilizing agents, plasticizers, acidulants, polymers, mucoadhesive agents, bioadhesive polymers, stabilizers, emulsifying agents, suspending agents, sweeteners, preservatives, flavoring agents, coloring agents, film-forming agents, mouth feel improvers and solvents and the like. Co-processed excipients are also covered under the scope of the present invention. The excipients may be in the form of a powder or the form of a dispersion. Combination of excipients performing the same function may also be used to achieve desired formulation characteristics. Excipients may be present in any part (intra and/or extra granular) of the composition in any proportion.

Unless the context requires otherwise, throughout the present specification and claims, the word "comprise" and variations thereof, such as, "comprises" and "comprising" are to be construed in an open, inclusive sense, that is as "including, but not limited to".

As used in this specification, the singular forms "a", "an", and "the" include plural references unless the context clearly dictates otherwise. Thus, for example, a reference to "a process" includes one or more processes, and/ or steps of the type described herein and/ or which will become apparent to those persons skilled in the art upon reading this disclosure. Unless otherwise stated the weight percentages expressed herein are based on the final weight of the composition. As used herein, the term "about" means ± approximately 20% of the indicated value, such that "about 10 percent" indicates approximately 08 to 12 percent.

As used herein, the term "intra-granular" (part/phase/portion) refers to the components of formulation of the present invention that are within granules. As used herein, the term "extra-granular" (part/phase/portion) refers to those components of the formulation of the present invention that are outside the granules.

The term "patient" and/or "subject" are used interchangeably herein. In some embodiments, the patient or subject is a human. In further embodiments, the patient or subject is an animal. In some embodiments, the human can be of any age such as adult, adolescent, pediatric or geriatric.

The term "viral infection" or "virus infection" or "infection" means an infection caused by a virus. The infection may be caused by any virus from filoviridae, flaviviridae, paramyxoviridae, orthomyxoviridae, coronaviridae, arenaviridae and/or adenoviridae families. Preferably, the infection is a respiratory viral infection. In one embodiment, the respiratory viral infection includes, but is not limited to, influenza viral infection including Influenza A, Influenza B, and Influenza C (seasonal flu), rhinovirus infection (common cold), coronavirus infection caused by alpha, beta, gamma, and delta virus families, coronavirus infection (Severe Acute Respiratory Syndrome and common cold), coronavirus disease 2019 (COVID-19), paramyxovirus infection (measles), choriomeningitis virus, junin virus, lassa virus, guanarito virus, sabia, lujo, lymphocytic choriomeningitis virus, machupo virus, chapare, Ebola virus, and Marburg virus.

As used herein, the term "therapeutic agent" means an agent or drug utilized to treat, combat, ameliorate, prevent or improve an unwanted condition or allergy or disease, or infection of a patient. The composition may be administered, as the case may be, before or after the onset of a viral infection, i.e. for prophylactic or therapeutic treatment purposes, or both. The term "therapeutically equivalent" means a formulation where its therapeutic effect is equivalent to a reference formulation.

Tₘₐₓ refers to the observed time to reach a maximum plasma concentration. Cₘₐₓ refers to the maximum plasma concentration. Cₘᵢₙ refers to the minimum plasma concentration. AUC_{0-inf} refers to the area under the curve in a plot of analyte concentration in blood plasma versus time from zero to infinity. AUC₀₋ₜ refers to the area under the curve in a plot of analyte concentration in blood plasma versus time from zero to a specified time. AUC₀₋ₜₐᵤ refers to the area under the concentration-time curve from time 0 to time of the last quantifiable concentration. T_{1/2} refers to the time required to eliminate one-half of the plasma concentration of an analyte. Kel refers to the elimination rate constant.

The term "stable" refers to the compositions of the present invention, wherein the amount of the active ingredient of a formulation does not deviate from the initial amount by more than the values given in the specification or the guidelines of the common Pharmacopoeias or loss in active ingredient is less than 50% (less than 40%, 30%, 20%, 10%, 5%) of the initial content after being stored for at least 1 month, preferably for at least 2 months, preferably for at least 3 months, more preferably for at least 6 months, more preferably for at least 12 months or more preferably for at least 24 months. The stability of the composition may be evaluated at "long-term" conditions of 25°C/60% RH, at "intermediate conditions" of 30°C/65% RH, at "accelerated conditions" of 40°C/75% RH, in the final container measured as the loss in the content of active ingredient. Stability testing may be conducted according to the current guidelines by ICH and FDA. In another embodiment of the invention, there is provided a sublingual tablet composition, wherein the total impurity is less than 5%, less than 3%, less than 2.5%, less than 2%, less than 1.5%, less than 1%, less than 0.9%, less than 0.8%, less than 0.7%, less than 0.6%, or less than 0.5%. In a preferred embodiment of the invention, the total impurity is less than 1%.

Total impurities may include **Compound A** (GS-711463/Phosphate RDV or [(2∼{R},3∼{S},4∼{R},5∼{R})-5-(4-azanylpyrrolo[2,1-f][1,2,4]triazin-7-yl)-5-cyano-3,4-bis(oxidanyl)oxolan-2-yl]methyl dihydrogen phosphate), and/or **Compound B** (Diastereomer/RDV-1 or 2-ethylbutyl ((R)-(((2R,3S,4R,5R)-5-(4-aminopyrrolo[2,1-f][1,2,4]triazin-7-yl)-5-cyano-3,4-dihydroxytetrahydrofuran-2-yl)methoxy)(phenoxy) phosphoryl)-L-alaninate), and/or **Compound C** (Triol nitrile impurity/GS-441524/RDV-3 or (2R,3R,4S,5R)-2-(4-Aminopyrrolo[2,1-f][1,2,4]triazin-7-yl)-3,4-dihydroxy-5-(hydroxymethyl)tetrahydrofuran-2-carbonitrile). Total impurities are controlled with a specification limit of NMT 2.5%.

The present invention relates to transmucosal compositions of antiviral drugs and one or more pharmaceutically acceptable excipients and processes for preparing such compositions.

The present invention relates to transmucosal compositions of antiviral drugs such as remdesivir, favipiravir, baloxavir marboxil, besifovir, raltegravir, molnupiravir, GS-441524, and ravidasvir, or any combinations thereof and one or more pharmaceutically acceptable excipients and processes for preparing such compositions.

The present invention also relates to transmucosal dosage forms like sublingual pharmaceutical composition comprising an antiviral drug such as remdesivir, favipiravir, baloxavir marboxil, besifovir, raltegravir, molnupiravir, GS-441524, and ravidasvir, and one or more pharmaceutically acceptable excipients selected from the group comprising diluent, binder, disintegrant, lubricant, glidant, surfactant, pH regulating agent, effervescent agent, salivating agent, solubilizing agent, stabilizer, sweetener, taste masking agent, preservative, flavoring agent, coloring agent, film-forming agent, and solvent or mixtures thereof.

The present invention relates to transmucosal compositions of remdesivir or its pharmaceutically acceptable salts or solvates thereof.

In one embodiment of the invention, there is provided a sublingual pharmaceutical composition comprising remdesivir or its pharmaceutically acceptable salts or solvates thereof and one or more pharmaceutically acceptable excipients and processes for preparing such compositions. In one embodiment of the invention, there is provided a sublingual pharmaceutical composition comprising molnupiravir and one or more pharmaceutically acceptable excipients and processes for preparing such compositions. In one embodiment of the invention, there is provided a sublingual pharmaceutical composition comprising favipiravir and one or more pharmaceutically acceptable excipients and processes for preparing such compositions.

The present invention also relates to a pharmaceutical composition, wherein the composition is formulated for rapid disintegration in sublingual administration.

In another embodiment of the invention, there is provided a transmucosal dosage form like sublingual pharmaceutical composition, which exhibit desired pharmaceutical technical attributes such as, pH, disintegration, dissolution, thickness, diameter, hardness, friability, compressibility index, assay, related substance, content uniformity, stability, bioequivalence, therapeutic equivalence, patient compliance, and commercial viability.

In another embodiment of the invention, there is provided a sublingual pharmaceutical composition comprising remdesivir or its pharmaceutically acceptable salts or solvates thereof and one or more pharmaceutically acceptable excipients, which exhibit desired pharmaceutical technical attributes such as wetting time, pH, disintegration, dissolution, thickness, diameter, hardness, friability, compressibility index, assay, related substance, content uniformity, stability, bioequivalence, therapeutic equivalence, patient compliance, and commercial viability.

The present inventors have performed extensive research with the aim of conceiving a transmucosal pharmaceutical composition of antiviral drugs, with the aim of obtaining a composition that shall be as effective as the known compositions in its preventive or curative effect, than the known compositions.

In consideration of the need, the inventors of the present invention have done extensive research and conducted several experiments to develop a sublingual pharmaceutical composition with desired formulation technical attributes. It was surprising to the inventors of the present invention that they could achieve quick tablet disintegration without leaving an unpleasant taste in the mouth. The present inventors were able to completely mask the bitter taste of the drug and control the pH range of the composition (from about 2.0 to about 6.5) which facilitate the dissolution of the drug in sublingual mucosa. The inventors surprisingly obtained the desired drug release in all physiological pH range (pH of 1.2 in 0. IN Hydrochloric Acid, pH 3.0 in McIlvaine Buffer, and pH 6.8 in Phosphate buffer) for the developed sublingual formulation which was achieved after carrying out diligent scientific experimentations and optimization of the formulation and process variables to achieve successful formulation such as using certain excipients in a certain ratio (a drug to pH regulating agents, taste masking agents, sweetening agents and/or flavoring agents and like), use of certain excipients in intra and/or extra part, and the like, while many other formulations outside the preview of the present invention did not provide desired formulation technical attributes.

In another embodiment of the invention, there is provided a sublingual pharmaceutical composition comprising remdesivir or its pharmaceutically acceptable salts or solvates thereof and one or more pharmaceutically acceptable excipients comprising diluent, binder, disintegrant, surfactant, pH regulating agent, glidant, lubricant, solubilizing agent, sweetener, taste masking agent, and flavoring agent.

In another embodiment of the invention, there is provided a sublingual pharmaceutical composition, wherein the composition is free of any binder. In another embodiment of the invention, there is provided a sublingual pharmaceutical composition, wherein the composition comprises binder from 0 to about 2%, preferably from 0 to about 1%.

In another embodiment of the invention, there is provided a sublingual pharmaceutical composition comprising: an intra-granular phase comprising an antiviral drug, one or more pharmaceutically acceptable excipients selected from the group comprising diluent, disintegrant, solubilizing agent, and pH regulating agent; and an extra-granular phase comprising one or more pharmaceutically acceptable excipients selected from diluent, disintegrant, sweetener, taste masking agent, flavoring agent, glidant and lubricant.

In another embodiment of the invention, there is provided a sublingual pharmaceutical composition comprising: an intra-granular phase comprising remdesivir or its pharmaceutically acceptable salts or solvates thereof, one or more pharmaceutically acceptable excipients selected from the group comprising diluent, disintegrant, solubilizing agent, and pH regulating agent; and an extra-granular phase comprising one or more pharmaceutically acceptable excipients selected from diluent, disintegrant, sweetener, taste masking agent, flavoring agent, glidant and lubricant.

In another embodiment of the invention, the disintegrant is present in both the intra and extra granular phases of the composition. In another embodiment, the disintegrant is equally divided in both the intra and extra granular phases of the composition.

In another embodiment of the invention, the pharmaceutical composition as per the present invention is a sublingual tablet. In another embodiment, the composition is in the form of buccal tablet dosage form.

In another embodiment of the invention, the pharmaceutical composition as per the present invention is a compressed sublingual tablet.

In another embodiment of the invention, the pharmaceutical composition as per the present invention provides a sublingual pharmaceutical composition, in which the bitter taste of antiviral drugs is masked by one or more taste masking agents. In another embodiment, the bitter taste of remdesivir is masked by one or more taste masking agents.

In another embodiment of the invention, there is provided a sublingual pharmaceutical composition with good patient compliance and acceptance, especially for children and elderly patients.

In one embodiment, pharmaceutical compositions of the present invention are the solid pharmaceutical compositions that rapidly disintegrate in the mouth of a subject, upon insertion into the buccal cavity or when placed under the tongue. In another embodiment of the invention, there is provided a sublingual tablet composition, wherein the sublingual tablet is intended to disintegrate in a time range of about 1 second to 360 seconds, preferably about 1 second to 240 seconds, preferably in about 1 second to 120 seconds or in about 1 second to 60 seconds or within about 30 seconds. Rapid disintegration may include where the pharmaceutical composition is disintegrated in about 1 second to 240 seconds, preferably in about 1 second to 120 seconds or in about 1 second to 60 seconds or within about 30 seconds. In another embodiment, the disintegration time is not more than 300 seconds. In another embodiment, the composition disintegrates and dissolves in the mouth after administration within 240 seconds. Disintegration time testing for tablets can be performed in a United States Pharmacopoeia 43 (USP) tablet disintegration tester wherein a tablet is placed in a basket, which moves upward and downward in a 1-liter beaker of water at 37±2° C.

In another embodiment of the invention, there is provided a sublingual tablet composition, wherein the composition exhibits intended wetting time to assess the capacity of the tablets to disintegrate by swelling in water in a time range of from about 1 second to 240 seconds.

In another embodiment of the invention, there is provided a sublingual tablet composition, wherein the composition has a pH from about 2.0 to about 7.0. In another embodiment of the invention, there is provided a sublingual tablet composition, wherein the composition has a pH from about 2.5 to about 7.0. In another embodiment of the invention, there is provided a sublingual tablet composition, wherein the composition has a pH from about 2.5 to about 6.5. In a preferred embodiment, the pH can be measured by the following method: disintegrate 1 tablet in 5 mL of purified water and record the pH using a calibrated pH meter.

In another embodiment of the invention, there is provided a transmucosal pharmaceutical composition in a solid dosage form comprising an antiviral drug in an amount of about 0.1 mg to about 50 mg and one or more pharmaceutically acceptable excipients, wherein the composition has a pH from about 2.0 to about 7.0, and the pH is measured by the following method: disintegrate 1 tablet in 5 mL of purified water and record the pH using a calibrated pH meter.

In another embodiment of the invention, there is provided a transmucosal pharmaceutical composition in a solid dosage form comprising an antiviral drug in an amount of about 0.1 mg to about 50 mg and one or more pharmaceutically acceptable excipients, wherein the composition has a pH from about 2.5 to about 7.0. In another embodiment, the composition is intended to dissolve within about 360 seconds in the mouth of a subject, preferably within 240 seconds upon placement under the tongue.

In another embodiment of the invention, there is provided a sublingual pharmaceutical composition comprising remdesivir or its pharmaceutically acceptable salts or solvates thereof in an amount of from about 0.1 mg to about 50 mg and one or more pharmaceutically acceptable excipients, wherein the composition has pH between about 2.0 and about 6.5 and is intended to dissolve in about 1 second to about 240 seconds in the mouth of a subject upon placement under the tongue.

In another embodiment of the invention, there is provided a pharmaceutical composition for sublingual administration comprising antiviral drug in an amount from about 0.1 mg to about 50 mg and at least one pH regulating agent, wherein the pH regulating agent is capable of maintaining the pH from about 2.0 to about 6.5 to facilitate dissolution of the drug in sublingual mucosa. In another embodiment, the pH regulating agent is capable of maintaining the pH from about 2.0 to about 4.0 to facilitate the dissolution of the drug in sublingual mucosa. The pH can be measured by the following method: disintegrate 1 tablet in 100 mL of purified water and record the pH using a calibrated pH meter. In a preferred embodiment, the pH can be measured by the following method: disintegrate 1 tablet in 5 mL of purified water and record the pH using a calibrated pH meter.

In another embodiment of the invention, there is provided a sublingual pharmaceutical composition comprising an antiviral drug and a pH regulating agent, wherein the ratio of the weight of the drug to the weight of the pH regulating agent is from 10:0.05 to 0.05:10.

The present invention further relates to sublingual pharmaceutical tablet compositions, wherein the drug is associated with cyclodextrin in the composition to improve the solubility profile of the drug.

In another embodiment of the invention, there is provided a sublingual pharmaceutical composition comprising an antiviral drug and a solubilizing agent, wherein the ratio of the weight of antiviral drug to the weight of the solubilizing agent is from 10:0.05 to 0.05:10. In another embodiment of the invention, there is provided a sublingual pharmaceutical composition comprising remdesivir or its pharmaceutically acceptable salts or solvates thereof and solubilizing agent, wherein the ratio of the weight of remdesivir to the weight of the solubilizing agent is from 10:0.05 to 0.05:10. In another preferred embodiment, the ratio of remdesivir to the solubilizing agent is 1:10 to 10:1. In another preferred embodiment, the ratio of remdesivir to the solubilizing agent is 1:4 to 10:1.

In another embodiment of the invention, there is provided a sublingual pharmaceutical composition comprising an antiviral drug and a taste masking agent, wherein the ratio of the weight of antiviral drug to the weight of the taste masking agent is from 10:0.05 to 0.05:10. In another embodiment of the invention, there is provided a sublingual pharmaceutical composition comprising remdesivir or its pharmaceutically acceptable salts or solvates thereof and taste masking agent, wherein the ratio of the weight of remdesivir to the weight of the taste masking agent is from 10:0.05 to 0.05:10. In another preferred embodiment, the ratio of remdesivir to the taste masking agent is 1:10 to 10:1. In another preferred embodiment, the ratio of remdesivir to the taste masking agent is 1:0.1 to 0.1:1. In another preferred embodiment, the ratio of remdesivir to the taste masking agent is 1:0.5 to 1:0.7. In another preferred embodiment, the ratio of remdesivir to the taste masking agent is 1:0.27. In another preferred embodiment, the ratio of remdesivir to the taste masking agent is 4:1.

In one embodiment, the drug is released 80% or more within 30 minutes or 20 minutes after administration of the composition. More preferably the composition releases at least 80% drug in 15 minutes or less. More preferably the composition releases not less than 85% drug in 5 minutes. More preferably the composition releases at least 75% drug in 15 minutes or less. The sublingual tablet compositions prepared by the process as per the present invention can be subjected to in vitro dissolution evaluation according to Test 711 "Dissolution" in the United States Pharmacopoeia 37, United States Pharmacopoeial Convention, Inc., Rockville, Md., 2014 ("USP") to determine the rate at which the active substance is released from the dosage form, and the content of the active substance can be determined in solution by high-performance liquid chromatography. In another embodiment, sublingual tablet compositions of the present invention exhibit at least 80% of drug release in 15 minutes or less in 900 ml of 0.1N Hydrochloric Acid, using a USP II apparatus (paddle) at a temperature of 37±0.5°C and a rotation speed of 50 revolutions per minute. In another embodiment, sublingual tablet compositions of the present invention exhibit at least 75% of drug release in 15 minutes or less in 900 ml of McIlvaine Buffer (pH 3.0), using a USP I apparatus (basket) at a temperature of 37±0.5°C and a rotation speed of 100 revolutions per minute. In another embodiment, sublingual tablet compositions of the present invention exhibit at least 75% of drug release in 15 minutes or less in 900 ml of Phosphate buffer (pH 6.8), using a USP II apparatus (paddle) at a temperature of 37±0.5°C and a rotation speed of 50 revolutions per minute.

In another embodiment of the invention, there is provided a transmucosal pharmaceutical composition in a solid dosage form comprising an antiviral drug in an amount of about 0.1 mg to about 100 mg and one or more pharmaceutically acceptable excipients, wherein the composition has a pH from about 2.5 to about 7.0 and the composition releases at least 75% of drug release in 15 minutes or less in 900 ml of McIlvaine Buffer (pH 3.0), using a USP I apparatus (basket) at a temperature of 37±0.5°C and a rotation speed of 100 revolutions per minute. In another embodiment of the invention, there is provided a transmucosal pharmaceutical composition in a solid dosage form comprising an antiviral drug in an amount of about 0.1 mg to about 50 mg and one or more pharmaceutically acceptable excipients, wherein the composition has a pH from about 2.5 to about 7.0 and the composition releases at least 75% of drug release in 15 minutes or less in 900 ml of McIlvaine Buffer (pH 3.0), using a USP I apparatus (basket) at a temperature of 37±0.5°C and a rotation speed of 100 revolutions per minute. In another embodiment of the invention, there is provided a transmucosal pharmaceutical composition in a solid dosage form comprising an antiviral drug in an amount of about 0.1 mg to about 50 mg and one or more pharmaceutically acceptable excipients, wherein the composition has a pH from about 2.5 to about 7.0 and the composition releases at least 75% of drug release in 15 minutes or less in 900 ml of Phosphate buffer (pH 6.8), using a USP II apparatus (paddle) at a temperature of 37±0.5°C and a rotation speed of 50 revolutions per minute.

In another embodiment of the invention, there is provided a pharmaceutical composition in the form of a tablet for sublingual administration comprising: an antiviral drug or its pharmaceutically acceptable salts or solvates thereof in an amount from about 0.1 mg to about 50 mg, a pH regulating agent capable of maintaining the pH from about 2.5 to about 7.0 to facilitate dissolution of the drug in sublingual mucosa, and one or more pharmaceutically acceptable excipients, wherein the composition releases at least 80% of the drug in 15 minutes or less when measured in 900 ml of 0.1N Hydrochloric acid using a USP II apparatus (Paddle) at a temperature of 37±0.5°C and a rotation speed of 50 revolutions per minute.

In another embodiment of the invention, there is provided a pharmaceutical composition in the form of a tablet for sublingual administration comprising: a) an antiviral drug selected from the group comprising remdesivir, favipiravir, baloxavir marboxil, besifovir, raltegravir, molnupiravir, GS-441524, and ravidasvir in an amount from about 0.1 mg to about 50 mg, b) a pH regulating agent capable of maintaining the pH from about 2.5 to about 7.0, c) a solubilizing agent, present in an amount such that the ratio of the weight of antiviral drug to the weight of the solubilizing agent is from 10:0.05 to 0.05:10 or 1:10 to 10:1 or preferably from 1:4 to 10:1, d) and one or more pharmaceutically acceptable excipients, wherein the composition releases at least 75% of the drug in 15 minutes or less.

In another embodiment of the invention, there is provided a pharmaceutical composition in the form of a tablet for sublingual administration comprising: a) an antiviral drug selected from the group comprising remdesivir, favipiravir, baloxavir marboxil, besifovir, raltegravir, molnupiravir, GS-441524, and ravidasvir in an amount from about 0.1 mg to about 100 mg, b) a pH regulating agent capable of maintaining the pH from about 2.5 to about 7.0, c) a solubilizing agent, present in an amount such that the ratio of the weight of antiviral drug to the weight of the solubilizing agent is from 10:0.05 to 0.05:10 or 1:10 to 10:1 d) and one or more pharmaceutically acceptable excipients, wherein the composition releases at least 75% of drug release in 15 minutes or less in 900 ml of McIlvaine Buffer (pH 3.0), using a USP I apparatus (basket) at a temperature of 37±0.5°C and a rotation speed of 100 revolutions per minute.

In another embodiment of the invention, there is provided a pharmaceutical composition in the form of a solid tablet for sublingual administration comprising: remdesivir or its pharmaceutically acceptable salts or solvates thereof in an amount from about 0.1 mg to about 50 mg, a pH regulating agent capable of maintaining the pH from about 2.0 to about 6.5 to facilitate dissolution of the remdesivir in sublingual mucosa, and one or more pharmaceutically acceptable excipients, wherein the composition releases at least 80% of the remdesivir in 15 minutes or less when measured in 900 ml of 0.1N Hydrochloric acid using a USP II apparatus (Paddle) at a temperature of 37±0.5°C and a rotation speed of 50 revolutions per minute. In another embodiment, sublingual tablet compositions exhibit at least 75% of drug release in 15 minutes or less in 900 ml of McIlvaine Buffer (pH 3.0), using a USP I apparatus (basket) at a temperature of 37±0.5°C and a rotation speed of 100 revolutions per minute. In another embodiment, sublingual tablet compositions exhibit at least 75% of drug release in 15 minutes or less in 900 ml of Phosphate buffer (pH 6.8), using a USP II apparatus (paddle) at a temperature of 37±0.5°C and a rotation speed of 50 revolutions per minute.

In another embodiment of the invention, there is provided a pharmaceutical composition in the form of a sublingual tablet pharmaceutical composition comprising remdesivir or its pharmaceutically acceptable salts or solvates thereof in an amount of about 0.1 mg to about 50 mg, and one or more pharmaceutically acceptable excipients including a taste masking agent to reduce the numbness of the mucosa present in the weight ratio of the remdesivir from 10:0.05 to 0.05:10, and wherein the composition has a pH from about 2.0 to about 4.0 and dissolves within about 120 seconds in the mouth of a subject upon placement under the tongue, and wherein said sublingual composition provides a pharmacokinetic profile substantially equivalent to the pharmacokinetic profile of intravenous injectable dosage form comprising remdesivir.

In another embodiment of the invention, there is provided a sublingual tablet pharmaceutical composition comprising remdesivir or its pharmaceutically acceptable salts or solvates thereof in an amount of about 0.1 mg to about 50 mg, and one or more pharmaceutically acceptable excipients including a solubilizing agent, present in an amount such that the ratio of the weight of remdesivir to the weight of the solubilizing agent is from 1:10 to 10:1, wherein said sublingual composition provides a pharmacokinetic profile substantially equivalent to the pharmacokinetic profile of intravenous injectable dosage form comprising remdesivir.

In another embodiment of the invention, the compositions prepared by the process as per the present invention have a hardness from about 1 to about 70 Newtons (N), and a friability of less than 2% when measured by USP (United States Pharmacopoeia) method. Preferably, the hardness of the tablet according to the present invention is between about 15 N to 30 N. More preferably, the hardness of the tablet according to the present invention is between about 20 N to 60 N.

In another embodiment of the invention, there is provided a sublingual tablet composition, wherein the composition has friability not more than 1%. Preferably, the friability of the tablet according to the present invention is not more than 1%. Several devices can be used to test tablet hardness such as a Monsanto tester, a Strong-Cobb tester, a Pfizer tester, an Erweka tester, a Schleuniger tester, etc. Friability can be determined using a Roche friabilator for 100 revolutions at 25 rpm (revolutions per minute).

In another embodiment of the invention, there is provided a sublingual tablet composition, which has a thickness of about 2 to about 10 mm. In another embodiment of the invention, there is provided a sublingual tablet composition, which has a diameter of about 2 to about 12 mm, preferably, 3 to 8 mm. In another embodiment of the invention, there is provided a sublingual tablet composition that has a Carr's index in the range of about 1% to 30%, preferably about 20% value, more preferably about 5% to 15% value, which indicates good flowability.

In another embodiment of the invention, there is provided a sublingual tablet composition, wherein the composition has an assay in the range from 90% to 110% as measured by HPLC (High-Performance Liquid Chromatography) method using a suitable column (such as Zorbax SB-C18).

In yet another embodiment, the sublingual compositions as described herein exhibits an assay amount of at least 98.0% after one to two months while stored at 40°±2°C and 75±5% relative humidity. In yet another embodiment, the sublingual compositions as described herein exhibits an assay amount of at least 98.0% after one to two months while stored at 25±2°C and 60±5% relative humidity.

In yet another embodiment, the sublingual compositions as described herein have an amount of **Compound A** of not more than 3.0% by HPLC after one-month period while stored at 25±2°C and 60±5% relative humidity and/or 40°±2°C and 75±5% relative humidity. In a preferred embodiment, the sublingual compositions as described herein have an amount of **Compound A** of not more than 0.5% by HPLC after one-month period while stored at 40°±2°C and 75±5% relative humidity. In another preferred embodiment, the sublingual compositions as described herein have an amount of **Compound A** of not more than 0.2% by HPLC after one-month period while stored at 40°±2°C and 75±5% relative humidity.

In yet another embodiment, the sublingual compositions as described herein have an amount of **Compound B** of not more than 1.5% by HPLC after one-month period while stored at 25±2°C and 60±5% and/or 40°±2°C and 75±5% relative humidity. In a preferred embodiment, the sublingual compositions as described herein have an amount of **Compound B** of not more than 0.4% by HPLC after one-month period while stored at 40°±2°C and 75±5% relative humidity. In another preferred embodiment, the sublingual compositions as described herein have an amount of **Compound B** of not more than 0.3% by HPLC after one-month period while stored at 40°±2°C and 75±5% relative humidity.

In yet another embodiment, the sublingual compositions as described herein have an amount of **Compound C** of not more than 2.0% by HPLC after one-month period while stored at 25±2°C and 60±5% and/or 40°±2°C and 75±5% relative humidity. In a preferred embodiment, the sublingual compositions as described herein have an amount of **Compound C** of not more than 0.2% by HPLC after one-month period while stored at 40°±2°C and 75±5% relative humidity. In another preferred embodiment, the sublingual compositions as described herein have an amount of **Compound C** of not more than 0.1% by HPLC after one-month period while stored at 40°±2°C and 75±5% relative humidity.

In yet another embodiment, the sublingual compositions as described herein have an amount of **total impurities** of not more than 2.0% by HPLC after one-month period while stored at 25±2°C and 60±5% relative humidity. In yet another embodiment, the packaged sublingual compositions as described herein have an amount of **total impurities** of not more than 2.0% by HPLC after one-month period while stored at 40°±2°C and 75±5% relative humidity.

In another embodiment of the invention, content uniformity of the sublingual composition is within acceptable limits and meets the requirements for dosage uniformity, in the acceptance value less than or equal to 15% as measured by a suitable technique such as HPLC (High-Performance Liquid Chromatography) method.

In one embodiment, the pharmaceutical composition may comprise antiviral drug in the range of about 0.1% to about 97% by weight, preferably in the range of about 5% to about 85% by weight, on the basis of the total weight of the composition. In another embodiment, the sublingual tablet composition comprises a) about 0.1% to about 99% of an antiviral drug, and b) about 0.1% to about 90% of one or more pharmaceutically acceptable excipients. In another embodiment, the sublingual tablet composition comprises: a) about 0.1% to about 97% of remdesivir or its pharmaceutically acceptable salts or solvates thereof, and b) about 0.1% to about 90% of one or more pharmaceutically acceptable excipients.

In another embodiment of the invention, the sublingual composition comprises: a) from about 0.1% to about 97% by weight of an antiviral drug, b) from 0 to about 80% by weight of one or more diluents, c) from 0 to about 30% by weight of one or more binders, d) from 0 to about 20% by weight of one or more disintegrants, e) from 0 to about 5% by weight of one or more surfactants, f) from 0 to about 5% by weight of one or more glidants, g) from 0 to about 20% by weight of one or more pH regulating agents, h) from 0 to about 40% by weight of one or more solubilizing agents, i) from about 0.1% to about 5% by weight of one or more lubricants, j) from about 0 to about 5% by weight of one or more preservatives, k) from 0 to about 5% by weight of one or more sweeteners, 1) from 0 to about 5% by weight of one or more flavor, and optionally, m) one or more other pharmaceutically acceptable excipients.

In another embodiment of the invention, the sublingual composition comprises: a) from about 0.1% to about 97% by weight of an antiviral drug, b) from 0 to about 80% by weight of one or more diluents, c) from 0 to about 30% by weight of one or more binders, d) from 0 to about 20% by weight of one or more disintegrants, e) from 0 to about 40% by weight of one or more solubilizing agents, f) from about 0 to about 5% by weight of one or more sweeteners, g) from 0 to about 10% by weight of one or more taste masking agents, h) from 0 to about 5% by weight of one or more flavoring agents, i) from 0 to about 5% by weight of one or more glidants, j) from about 0.1% to about 5% by weight of one or more lubricants.

In another embodiment of the invention, the sublingual composition comprises: a) from about 0.1% to about 97% by weight of remdesivir or its pharmaceutically acceptable salts or solvates thereof, b) from 0 to about 70% by weight of one or more diluents, c) from 0 to about 30% by weight of one or more binders, d) from 0 to about 20% by weight of one or more disintegrants, e) from 0 to about 50% by weight of one or more carriers, f) from 0 to about 5% by weight of one or more surfactants, g) from 0 to about 5% by weight of one or more glidants, h) from 0 to about 5% by weight of one or more pH regulating agents, i) from 0 to about 30% by weight of one or more solubilizing agents, j) from about 0.1% to about 5% by weight of one or more lubricants, k) from 0 to about 5% by weight of one or more preservatives, 1) from about 0.1% to about 5% by weight of one or more sweeteners, m) from 0 to about 20% by weight of one or more bio/mucoadhesive agents, and optionally, n) one or more other pharmaceutically acceptable excipients.

In another embodiment of the invention, the sublingual tablet composition comprises: a) from about 0.1% to about 30% by weight of an antiviral drug, b) from about 20% to about 70% by weight of one or more diluents, c) from about 0.1% to about 5% by weight of one or more binders, d) from about 0.1% to about 10% by weight of one or more disintegrants, e) from about 0.01% to about 35% by weight of one or more solubilizing agent, f) from about 0.01% to about 3% by weight of one or more glidants, g) from about 0.01% to about 8% by weight of one or more pH regulating agents, h) from about 0.01% to about 2% by weight of one or more lubricants, i) from about 0.01% to about 3% by weight of one or more sweeteners, and optionally j) one or more other pharmaceutically acceptable excipients.

In another embodiment of the invention, the sublingual tablet composition comprises: a) from about 0.1% to about 35% by weight of an antiviral drug, b) from about 20% to about 70% by weight of one or more diluents, c) from about 0.1% to about 10% by weight of one or more disintegrants, d) from about 0.01% to about 35% by weight of one or more solubilizing agents, e) from about 0.01% to about 10% by weight of one or more sweeteners, f) from about 0.01% to about 5% by weight of one or more taste masking agents, g) about 0.01% to about 5% by weight of one or more flavoring agents, and h) one or more pH regulating agents.

In another embodiment of the invention, the sublingual tablet composition comprises: a) from about 0.1% to about 35% by weight of remdesivir or its pharmaceutically acceptable salts or solvates thereof, b) from about 20% to about 70% by weight of one or more diluents, c) from about 0.1% to about 5% by weight of one or more binders, d) from about 0.1% to about 10% by weight of one or more disintegrants, e) from about 0.01% to about 3% by weight of one or more surfactants, f) from about 0.01% to about 3% by weight of one or more glidants, g) from about 0.01% to about 5% by weight of one or more pH regulating agents, h) from about 0.01% to about 3% by weight of one or more lubricants, i) from about 0.01% to about 5% by weight of one or more sweeteners, and optionally j) one or more other pharmaceutically acceptable excipients.

In another embodiment of the invention, the sublingual composition comprises: a) from about 0.1% to about 85% by weight of remdesivir or its pharmaceutically acceptable salts or solvates thereof, b) from about 20% to about 70% by weight of one or more diluents, c) from about 0.1% to about 10% by weight of one or more binders, d) from about 0.1% to about 15% by weight of one or more disintegrants, e) from about 0.01% to about 3% by weight of one or more glidants, f) from about 0.01% to about 20% by weight of one or more solubilizing agents, g) from about 0.01% to about 3% by weight of one or more lubricants, h) from about 0.01% to about 5% by weight of one or more sweeteners, and optionally i) one or more other pharmaceutically acceptable excipients.

In another embodiment of the invention, the sublingual tablet composition comprises: a) from about 0.1% to about 35% by weight of remdesivir or its pharmaceutically acceptable salts or solvates thereof, b) from about 20% to about 70% by weight of one or more diluents, c) from about 0.1% to about 5% by weight of one or more binders, d) from about 0.1% to about 10% by weight of one or more disintegrants, e) from about 0.01% to about 3% by weight of one or more glidants, f) from about 0.01% to about 20% by weight of one or more solubilizing agents, g) from about 0.01% to about 3% by weight of one or more lubricants, h) from about 0.01% to about 5% by weight of one or more sweeteners, and optionally i) one or more other pharmaceutically acceptable excipients.

In another embodiment of the invention, the sublingual tablet composition comprises: a) from about 0.1% to about 35% by weight of remdesivir or its pharmaceutically acceptable salts or solvates thereof, b) from about 20% to about 70% by weight of one or more diluents, c) from about 0.1% to about 10% by weight of one or more disintegrants, d) from about 0.01% to about 35% by weight of one or more solubilizing agents, e) from about 0.01% to about 10% by weight of one or more sweeteners, f) from about 0.01% to about 5% by weight of one or more taste masking agents, g) about 0.01% to about 5% by weight of one or more flavoring agents, h) from about 0.01% to about 3% by weight of one or more glidants, i) from about 0.01% to about 3% by weight of one or more lubricants, and j) one or more pH regulating agents.

In another embodiment of the invention, the sublingual tablet composition comprises an intra-granular phase comprising: a) from about 0.1% to about 35% by weight of remdesivir or its pharmaceutically acceptable salts or solvates thereof, b) from about 20% to about 30% by weight of one or more diluents, c) from about 0.1% to about 5% by weight of one or more disintegrants, d) from about 0.01% to about 35% by weight of one or more solubilizing agents, and e) one or more pH regulating agents, and an extra-granular phase comprising: a) from about 20% to about 30% by weight of one or more diluents, b) from about 0.01% to about 10% by weight of one or more sweeteners, c) from about 0.01% to about 5% by weight of one or more taste masking agents, d) about 0.01% to about 5% by weight of one or more flavoring agents, e) from about 0.01% to about 3% by weight of one or more glidants, and f) from about 0.01% to about 3% by weight of one or more lubricants. The weight percentages are based on the total weight of the composition.

In another embodiment of the invention, the sublingual tablet composition comprises an intra-granular phase comprising: a) from about 0.1% to about 35% by weight of remdesivir or its pharmaceutically acceptable salts or solvates thereof, b) from about 20% to about 30% by weight of microcrystalline cellulose, c) from about 0.1% to about 5% by weight of crospovidone, d) from about 0.01% to about 35% by weight of beta-cyclodextrin, and e) one or more pH regulating agents selected from Hydrochloric acid and Sodium hydroxide, and an extra-granular phase comprising: a) from about 20% to about 30% by weight of microcrystalline cellulose, b) from about 0.01% to about 10% by weight of thaumatin, c) from about 0.01% to about 5% by weight of taste masker, d) about 0.01% to about 5% by weight of peppermint flavor, e) from about 0.01% to about 3% by weight of colloidal silicon dioxide, and f) from about 0.01% to about 3% by weight of magnesium stearate. The weight percentages are based on the total weight of the composition.

In another embodiment of the invention, the sublingual tablet composition comprises: a) about 6.7% by weight of remdesivir or its pharmaceutically acceptable salts or solvates thereof, b) about 50% by weight of one or more diluents, c) about 6.5% by weight of one or more disintegrants, d) about 26.6% by weight of one or more solubilizing agents, e) about 8.2% by weight of one or more sweeteners, taste masking agents, and flavoring agents, f) about 1% by weight of one or more glidants, g) about 1% by weight of one or more lubricants, and h) one or more pH regulating agents.

In another embodiment of the invention, the diluent comprises one or more of mannitol, lactose, microcrystalline cellulose, silicified microcrystalline cellulose, sucrose, starch, pregelatinized starch, xylitol, sorbitol, maltodextrin, polydextrose, isomalt, or mixtures thereof.

In another embodiment of the invention, the pH regulating agent comprises one or more organic acids (such as citric acid), inorganic acids (such as hydrochloric acid, sulphuric acid, phosphoric acid, or hydrobromic acid), organic bases, inorganic bases (sodium hydroxide), amino acids or mixtures thereof. In a preferred embodiment, the pH regulating agent comprises one or more of citric acid, tartaric acid, oxalic acid, hydrochloric acid, sodium hydroxide, or mixtures thereof. In a more preferred embodiment, the pH regulating agents comprise hydrochloric acid and sodium hydroxide. These pH regulating agents are capable of maintaining the pH of the composition from about 2.5 to about 7.0.

In another embodiment of the invention, one or more solubilizing agents are selected from the group comprising cyclodextrin (cyclodextrin analogs, such as but not limited to, alpha, beta- and gamma-cyclodextrin analogs and their derivatives), sodium lauryl sulfate, sodium carboxymethylcellulose, povidone, or mixtures thereof. In a preferred embodiment, the solubilizing agent is betadex sulfobutyl ether sodium.

In another embodiment of the invention, one or more taste masking agents are selected from the group comprising amberlite, polymethacrylates, taste-masker, sodium starch glycolate, carbopol polymers, sodium acetate, ethylcellulose, and polyvinyl acetate dispersion, trehalose, vinylacetate, polystyrene, cellulose acetate butyrate, or mixtures thereof.

In another embodiment of the invention, one or more sweeteners or sweetening agents are selected from the group comprising sucrose, sucralose, glucose, dextrose, saccharin sodium, aspartame, acesulfame potassium, neohesperidine dihydrochalcone, mannitol, xylitol, and thaumatin, and mixtures thereof.

In another embodiment of the invention, one or more flavoring agents are selected from the group comprising menthol, orange, grape, cherry, bubble gum flavor, tutti-frutti flavor, peppermint flavor, bitter taste masker flavor, and mixtures thereof.

In another embodiment of the invention, there is provided an antiviral sublingual composition, wherein the drug has a particle size distribution D₉₀ less than about 200 µm, D₅₀ less than about 100 µm, and D₁₀ less than about 50 µm. In another embodiment of the invention, there is provided a sublingual pharmaceutical composition comprising remdesivir or its pharmaceutically acceptable salts or solvates thereof and one or more pharmaceutically acceptable excipients, wherein remdesivir has a particle size distribution D₉₀ less than about 200 µm, D₅₀ less than about 100 µm and D₁₀ less than about 50 µm.

In another embodiment of the invention, the sublingual pharmaceutical composition is prepared by wet granulation (rapid mixture granulation, fluid bed granulation, spray drying), dry granulation, dry blending, dry mixing, and direct compression. Other formulation techniques are also contemplated within the scope of the present invention such as extrusion-spheronization, hot-melt extrusion, freeze-drying, spray drying, mass extrusion, and molding. In a preferred embodiment of the present invention, the composition is prepared by a wet granulation process in the presence of one or more solvents.

In another embodiment of the invention, the solvent used in the preparation of the composition is selected from the group comprising an aqueous solvent, alcoholic solvent, hydroalcoholic solvent, acidic solvent, bases, an ether solvent, or any mixtures thereof. In a preferred embodiment, one or more solvents used in the preparation of the composition are selected from the group comprising ethanol, water, tetrahydrofuran, hydrochloric acid, sodium hydroxide, or any mixtures thereof in any volume ratio. More preferably, the solvents comprise ethanol and purified water. In one embodiment, the solvent is a mixture of ethanol and hydrochloric acid (preferably IN hydrochloric acid) in 50:50 ratios.

In another embodiment of the invention, there is provided a process of preparing sublingual tablet compositions that includes the steps of a) sifting the accurately weighed quantities of one or more pharmaceutically acceptable excipient(s) such as diluent, disintegrant, glidant, and binder through a suitable sieve, b) preparing a solution or dispersion of drug and solubilizing agent in a suitable solvent, c) granulating the mixture of step a) with a solution or dispersion of step b), d) drying the granulated mass, optionally milling of the dried granules, e) blending of dried granules with one or more pharmaceutically acceptable excipient(s) such as suitable diluent, disintegrant, glidant, pH regulating agent, sweetener, flavor, and lubricant and passing through a suitable sieve, and f) compressing the lubricated granules into tablets.

In another embodiment of the invention, there is provided a process of preparing a sublingual tablet composition that includes the steps of: a) weighing all the ingredients separately, b) sifting the accurately weighed quantities of one or more pharmaceutically acceptable excipient(s) such as one or more of diluent, and disintegrant through a suitable sieve (such as ASTM #30), c) preparing a solution of the solubilizing agent in a suitable solvent (such as a mixture of purified water and ethanol), followed by addition of drug, followed by addition of IN hydrochloric acid to get a suitable pH, and followed by addition of IN Sodium Hydroxide solution to adjust the suitable pH of the solution, d) granulating the mixture of step b) with the solution of step c), e) drying the wet granules, and sifting through a suitable sieve (such as ASTM #30), f) blending of dried granules with suitable excipients such as diluent, disintegrant, sweetener, taste masking agent, flavoring agent, glidant, and lubricant and passing through a suitable sieve (such as ASTM #30), and g) compressing the lubricated granules into tablets.

In another embodiment of the invention, there is provided a sublingual pharmaceutical composition, wherein the composition is stable. In another embodiment, the composition is stable for at least one month while stored at 40°±2°C and 75±5% relative humidity.

In another embodiment, the present invention includes a sublingual pharmaceutical composition, wherein the composition is free of other polymorphic forms or any polymorphic form conversion.

In another embodiment of the invention, the pH regulating agent is selected such that the degradation of active ingredient or other components of the composition is slowed or reduced, as compared to a composition in which the pH regulating agent is not present. For example, the pH regulating agent may at least help in preventing significant degradation of the composition after a certain period of shelf life. The present inventors have surprisingly observed that the sublingual composition is stable in a pH range from about 2 to about 6.5. The present inventors have further observed that at pH higher than 6.5 (such as at pH 7, 7.5, or above) there was a significant increase (more than 10%) in impurities in the composition due to instability of the active ingredient.

In one embodiment, the sublingual pharmaceutical compositions as per the present invention provide a method of increasing the bioavailability of antiviral drugs such as remdesivir. More particularly, sublingual pharmaceutical compositions as per the present invention allow the active agent to bypass the first-pass metabolism, thereby enhancing the bioavailability of the active agent. Such sublingual compositions can offer several advantages over other modes of drug delivery, including, but not limited to, increased chemical stability of the active ingredient, sufficient shelf-life, good pharmacotechnical properties, stability, drug release, increasing the onset of action, lowering the required dosage, enhancing the efficacy and improving the safety profile of the active agent.

The present inventors were also able to achieve a pleasant mouth-feel for the developed sublingual dosage form, which disintegrates fast without leaving an unpleasant taste in the mouth. Remdesivir is a bitter-tasting drug. To increase the patient acceptability of the sublingual tablet compositions, the bitterness of the drug needs to be masked by using a suitable combination of one or more sweeteners, taste-masking agents, and/or flavoring agents. Organoleptic properties of the sublingual tablet compositions were evaluated for texture, taste, after taste, odor, flavor, and acceptability. The sublingual tablet compositions of Example 15, 16, 17 shown below in examples comprise thaumatin, bitter masker, and/or peppermint flavor and were perceived to have good overall acceptability based on tested organoleptic properties. In one embodiment of the invention, the ratio of the weight of remdesivir to the weight of the sweeteners is from 10:0.05 to 0.05:10. In another embodiment of the invention, the ratio of the weight of remdesivir to the weight of the sweeteners is 1:0.7. In one embodiment of the invention, the ratio of the weight of remdesivir to the weight of the flavoring agents is from 10:0.05 to 0.05:10. In another embodiment of the invention, the ratio of the weight of remdesivir to the weight of the flavoring agents is 1:0.27.

Bioavailability refers to the proportion of the drug administered that reaches the physiological site where the drug exerts its therapeutic effect, which is generally regarded as the bloodstream for many drugs. The bioavailability of a drug is most readily expressed as the concentration of the drug or its active metabolites in the blood plasma integrated over time. This quantity is commonly referred to as the "area under the curve" or "AUC". Sublingual administration according to this invention preferably increases the drug's bioavailability at least 5% or more, 10% or more, 20% or more, 30% or more, 40% or more, or 50% or more as compared to oral administration. In a further embodiment, relative bioavailability studies are performed to compare the bioavailability of the sublingual tablet formulation with an injection (intravenous) dosage form.

In one embodiment, the sublingual pharmaceutical compositions as per the present invention are bioequivalent and/or therapeutically equivalent to the reference product which is an injection dosage form. It may refer to one type of dosage form of a certain dose, e.g., a sublingual dosage form comprising about 0.1 mg to about 50 mg of the drug, having the same rate and extent of drug delivery as another type of dosage form at a certain dose, e.g., an injection dosage form, preferably an intravenous (IV) injection and can be shown by any method known in the art of pharmacodynamics or pharmacokinetics, and includes, but is not limited to, studies demonstrating that there is no significant difference between one type of dosage form and another type of dosage form for the mean maximal drug concentration (C), the area under the drug concentration-time curve (AUC), or the time to maximum concentration in the blood (T). In one embodiment, the compositions show comparative pharmacokinetics exposure in terms of point estimates which falls between 80%-125%. In certain embodiments, each remdesivir sublingual dosage form is bioequivalent and/or therapeutically equivalent to about 100 mg to 200 mg dose of remdesivir administered by intravenous (IV) administration.

The pharmacokinetic parameters are established by observing: a) a 90% Confidence Interval between 80% and 125% for the ratio (of Test Product vs. Reference Product) of the geometric mean of AUC₀₋ₜ, of GS-441524, b) a 90% Confidence Interval between 80% and 125% for the ratio (of Test Product vs. Reference Product) of the geometric mean AUC_{0-inf} of GS-441524, c) a 90% Confidence Interval between 80% and 125% for the ratio (of Test Product vs. Reference Product) of the geometric mean Cₘₐₓ of GS-441524, d) a combination of any of a) to c).

In one embodiment, the sublingual tablets are bioequivalent to an IV injection composition (such as intravenous infusion of remdesivir as a reference) when administered to an adult human under fasted conditions. In one embodiment, the sublingual tablets are bioequivalent to an IV injection composition (such as intravenous infusion of remdesivir as reference) when administered to an adult human under fed conditions.

In one embodiment, the sublingual tablet pharmaceutical composition comprises remdesivir or its pharmaceutically acceptable salts or solvates thereof in an amount of about 0.1 mg to about 50 mg, and one or more pharmaceutically acceptable excipients, wherein the composition is for at least 20 mg to 200 mg per day administration for a treatment duration of at least three days and wherein said composition, when administered to a human subject, provides a value of AUC₀₋ₜ for GS-441524 test to reference least squares mean ratio where the 90% confidence interval is between 80% and 125% of the natural-log transformed AUC₀₋ₜ value obtained with said commercially available remdesivir injection, and a value of AUC_{0-inf} GS-441524 test to reference least squares mean ratio where the 90% confidence interval is between 80% and 125% of the natural-log transformed AUC_{0-inf} value obtained with said commercially available remdesivir injection.

In one embodiment, the composition exhibits a Cₘₐₓ of from about 40 ng/mL to about 260 ng/mL for GS-441524, following administration of the sublingual composition to an adult human under fasted conditions. In one embodiment, the composition exhibits a Cₘₐₓ of from about 107 ng/mL to about 217 ng/mL for GS-441524, following administration of the sublingual composition to an adult human under fasted conditions in single-dose testing. In one embodiment, the composition exhibits a Cₘₐₓ of about 162.0±55.4 ng/mL. In one embodiment, the composition exhibits a Cₘₐₓ of from about 140 ng/mL to about 260 ng/mL for GS-441524, following administration of the sublingual composition to an adult human under fasted conditions in multiple-dose testing. In one embodiment, the composition exhibits a Cₘₐₓ of about 203.0±53.3 ng/mL.

In one embodiment, the composition exhibits an AUC₀₋ₜ of from about 800 ng.hr/mL to about 2400 ng.hr/mL for GS-441524, following administration of the sublingual composition to an adult human under fasted conditions. In one embodiment, the composition exhibits an AUC₀₋ₜ of from about 1100 ng.hr/mL to about 2300 ng.hr/mL for GS-441524, following administration of the sublingual composition to an adult human under fasted conditions in single-dose testing. In one embodiment, the composition exhibits an AUCo-tof about 1706.0±594.3 ng.hr/mL. In one embodiment, the composition exhibits an AUC_{0-inf} of from about 800 ng.hr/mL to about 2500 ng.hr/mL for GS-441524, following administration of the sublingual composition to an adult human under fasted conditions. In one embodiment, the composition exhibits an AUC_{0-inf} of from about 1100 ng.hr/mL to about 2400 ng.hr/mL for GS-441524, following administration of the sublingual composition to an adult human under fasted conditions in single-dose testing. In one embodiment, the composition exhibits an AUC_{0-inf} of about 1756.6±603.5 ng.hr/mL.

In one embodiment, the composition exhibits a Cₜₐᵤ of from about 30 ng/mL to about 90 ng/mL for GS-441524, following administration of the sublingual composition to an adult human under fasted conditions in multiple-dose testing. In one embodiment, the composition exhibits a Cₜₐᵤ of from about 40 ng/mL to about 80 ng/mL for GS-441524, following administration of the sublingual composition to an adult human under fasted conditions in multiple-dose testing. In one embodiment, the composition exhibits an Cₜₐᵤ of about 60.9±17.1 ng.hr/mL.

In one embodiment, the composition exhibits a Cₘᵢₙ of from about 25 ng/mL to about 80 ng/mL for GS-441524, following administration of the sublingual composition to an adult human under fasted conditions in multiple-dose testing. In one embodiment, the composition exhibits a Cₘᵢₙ of from about 35 ng/mL to about 65 ng/mL for GS-441524, following administration of the sublingual composition to an adult human under fasted conditions in multiple-dose testing. In one embodiment, the composition exhibits a Cₘᵢₙ of about 50.6±11.7 ng.hr/mL.

In one embodiment, the composition exhibits an AUC₀₋ₜₐᵤ of from about 1500 ng.hr/mL to about 3100 ng.hr/mL for GS-441524, following administration of the sublingual composition to an adult human under fasted conditions in multiple-dose testing. In one embodiment, the composition exhibits an AUC₀₋ₜₐᵤ of from about 1900 ng.hr/mL to about 2900 ng.hr/mL for GS-441524, following administration of the sublingual composition to an adult human under fasted conditions in multiple-dose testing. In one embodiment, the composition exhibits an AUC₀₋ₜₐᵤ of about 2413.9±475.1 ng.hr/mL.

In one embodiment, the composition of the present invention provides a method for achieving an AUC₀₋ₜₐᵤ of ≥1900 ng·hr/mL GS-441524, comprising administering daily to a human patient an effective amount of remdesivir sublingual compositions as per the present invention.

In one embodiment, the composition provides a value of AUCₜ for GS-441524 test to reference least squares mean ratio where the 90% confidence interval is between 80% and 125% of the natural-log transformed AUC₀₋ₜ value obtained with said commercially available remdesivir injection, and a value of AUC_{0-inf} GS-441524 test to reference least squares mean ratio where the 90% confidence interval is between 80% and 125% of the natural-log transformed AUC_{0-inf} value obtained with said commercially available remdesivir injection. The geometric mean ratios of the peak exposure and the extent of exposure obtained for GS-441524 at steady state with the intravenous and the sublingual formulation were found to be contained within 80-125%.

In one embodiment, the present invention provides use of sublingual pharmaceutical compositions of the present invention in methods for treating, preventing, ameliorating, and/or delaying the onset of one or more symptoms associated with or resulting from a viral infection, preferably a respiratory viral infection in a subject, caused by members of the filoviridae, flaviviridae paramyxoviridae, orthomyxoviridae, coronaviridae, arenaviridae or adenoviridae families. In an embodiment, the viral infection is a coronavirus infection. In another embodiment, the present invention provides the use of sublingual pharmaceutical compositions of the present invention in the treatment of coronavirus disease 2019 (COVID-19) infection. In another embodiment, the present invention provides the use of sublingual pharmaceutical compositions of the present invention in the treatment of moderate coronavirus disease 2019 (COVID-19) infection.

In certain embodiments, the methods provided herein comprise co-administering one or two or more antiviral active drugs as per present invention with an anti-viral vaccine, other antiviral drugs, antibiotics, antifungals, anti-inflammatories, protease inhibitors, JAK inhibitors, or other nucleoside or non-nucleoside antiviral drugs, which can be dosed orally, sublingually, buccally, intranasally, intravenously, and/or subcutaneously.

In certain embodiments, the subject or patient is a human. In certain embodiments, the subject or patient is an adult. In certain embodiments, the subject or patient is an adolescent. In certain embodiments, the subject or patient is a child. In one embodiment, the subject or patient is of age group between one-year-old to 90 years old. In one embodiment, the sublingual compositions of the present invention can be given to diabetic patients also because the composition is free of sugar.

In one embodiment, the symptoms or complications associated with viral infection or respiratory infection include, but are not limited to, chill, fever (mild or high fever), headache, sweating, fatigue, tiredness, sneezing, dry cough, sore throat, runny nose, vomiting, dehydration, cough with mucus, body aches, abdominal pain, joint pain, chest pain or discomfort, diarrhea, breathing problem or shortness, pneumonia, asthma, bronchitis, chronic obstructive pulmonary disease, congestive heart failure, mucormycosis, and/or any other symptoms or complications provided herein elsewhere.

In one embodiment, the composition can be administered once daily or divided into multiple daily doses from once per day to once in six months such as twice daily, three times daily, four times daily, five times daily, once a week, twice in a week, thrice in a week, once in two weeks, once in three weeks, once in four weeks or the like. In one embodiment, the composition is intended for at least once daily administration. In another embodiment, the composition is administered at least three times a day. In another embodiment, the composition is administered at least five times a day.

In one embodiment, for patients for whom the rapid onset of antiviral effect is required, the sublingual tablets in a sufficient amount to provide 5 mg to 400 mg per day of antiviral drugs can be administered as needed. In one embodiment, for patients for whom the rapid onset of antiviral effect is required, the sublingual tablets in a sufficient amount to provide 20 to 400 mg per day of remdesivir or its pharmaceutically acceptable salt thereof can be administered as needed. In a preferred embodiment, the sublingual tablets in a sufficient amount to provide 20 to 200 mg per day of remdesivir or its pharmaceutically acceptable salt thereof can be administered as needed. In another embodiment, the composition is self-administered by the patients to provide a daily dose from about 20 mg to about 400 mg of remdesivir. In another embodiment, the composition is self-administered by the patients to provide a daily dose from about 20 mg to about 200 mg of remdesivir. In another embodiment, the dose of the sublingual pharmaceutical composition of remdesivir to be administered is at least 20 mg for 5 times a day for at least five days. In another embodiment, the dose of the sublingual pharmaceutical composition of remdesivir to be administered is at least 100 mg once a day for a treatment duration of at least five days. In another embodiment, the dose of the sublingual pharmaceutical composition of remdesivir to be administered is at least 100 mg twice a day (b.i.d.) for a treatment duration of at least five days.

Yet another embodiment is a regimen for the treatment of viral infection including COVID-19 infection, which comprises administering a subject in need thereof, at least a loading dose of 200 mg to 400 mg followed by 200 mg to 400 mg maintenance dose and thereafter, in the form of sublingual tablets of any embodiment described herein.

In another embodiment, the sublingual pharmaceutical compositions as per the present invention are for self-administration by patients. In another embodiment, the sublingual pharmaceutical compositions as per the present invention provide more convenient and easy-to-administer dosage administration. In another embodiment, the sublingual pharmaceutical compositions as per the present invention eliminate the hospitalization requirements. In another embodiment, the sublingual pharmaceutical compositions as per the present invention are meant for pre-hospitalization use. In another embodiment, sublingual pharmaceutical compositions as per the present invention avoid requirements of specialized infrastructure set-up, hospital settings, trained professional personnel, and any special storage conditions, and accordingly are cost-efficient.

In one embodiment, the present invention further provides a method of treating COVID-19 infection, comprising: a) determining the glomerular filtration rate (eGFR) of a patient in need of treatment, b) administering the sublingual composition to the patient, if the eGFR is less than 30 mL/ min.

In another embodiment, the present invention further provides a method of treating COVID-19 infection, comprising: a) performing the hepatic laboratory testing in a patient in need of treatment, and b) consider discontinuing the treatment if ALT (alanine transaminase) levels increase to greater than 10 times the upper limit of normal level or if ALT elevation is accompanied by signs or symptoms of liver inflammation.

In another embodiment, the present invention further provides a method of treating COVID-19 infection, comprising: determining prothrombin time in all patients before starting the treatment and monitoring while receiving the treatment as clinically appropriate.

In one embodiment of the invention, the sublingual compositions as per the present invention provide an effective therapeutic effect against all coronavirus mutants including, but not limited to, B.1.351, B.1.1.7, N440K, E484Q, B.1.36, and B.1.617 variant, and others.

In another embodiment, sublingual pharmaceutical compositions of the present invention may be packaged in HDPE bottles or glass bottles, or blister packs. Packaging material may optionally contain desiccants. The packaging material used according to the present invention favorably protects the composition from air, light, and moisture. The packaging may be provided with a child-resistant closure. Preferably, the sublingual pharmaceutical compositions of the present invention are packaged in aluminum blister. Further, the composition of the present invention is packaged into a suitable package together with instructions to place the dosage form under a patient's tongue or in the buccal cavity. In one embodiment, the package also instructs the patients to allow the composition to dissolve completely after sublingual administration. In one embodiment, the package also instructs the patients not to eat and/or drink during and for a period of at least 5 to 10 minutes after administration of each sublingual dosage form.

Any pharmaceutically acceptable granulating agent can be used for wet granulation. Preferable granulating solvents include, but are not limited to, aqueous, non-aqueous, alcoholic, hydro-alcoholic, ether, acidic solvents, bases as a granulating liquid such as water, esters such as ethyl acetate, ketones such as acetone, alcohols such as methanol, ethanol (including ethanol 95%), isopropanol, butanol, dichloromethane, chloroform, dimethylacetamide, dimethyl sulfoxide, isopropyl acetate, ether (such as tetrahydrofuran, methyl tert-butyl ether, 1,4-dioxane and combinations thereof), diethyl ether, hydrochloric acid (such as IN hydrochloric acid), sodium hydroxide, mixtures of one or more alcohols and water and any combinations or mixtures thereof. In a further embodiment, solvents used as per the present invention are mixtures of one or more alcohol and water, in various volume ratios. In some embodiment, suitable organic or inorganic acids selected from the group comprising citric acid, malic acid, fumaric acid, tartaric acid, and hydrochloric acid can also be added in the granulation process or granulation solution. In one embodiment, the solvent is a mixture of ethanol and hydrochloric acid (preferably IN hydrochloric acid) in 50:50 ratios. In a preferred embodiment, the solvent is ethanol and/or water and mixtures thereof.

Various useful fillers or diluents include, but are not limited to, microcrystalline cellulose ("MCC"), sodium alginate, silicified MCC, microfine cellulose, lactitol, cellulose acetate, kaolin, glucose, lactose, maltose, fructose, sucrose, trehalose, starch, pregelatinized starch (PGS), sugar alcohols such as mannitol, D-mannitol, xylitol, maltitol, sorbitol, dextrates, dextrin, maltodextrin, compressible sugar, confectioner's sugar, dextrose, polydextrose, simethicone, calcium carbonate, calcium sulfate, calcium phosphate, dibasic calcium phosphate, tribasic calcium phosphate, magnesium carbonate, magnesium oxide, isomalt, a mixture of microcrystalline cellulose and mannitol or any mixtures thereof. The amount of diluent according to the present invention ranges from 0 to about 90% by weight of the composition. In an embodiment, the diluent according to the present invention is present in an amount of about 90% or less, 80% or less, e.g. 70% or less, 60% or less, 50% or less, 40% or less, 30% or less, 20% or less. In a preferred embodiment, the diluent according to the present invention is present in an amount of about 10% to about 80% by weight of the composition.

Various useful binders include, but are not limited to, acacia, guar gum, xanthan gum, alginic acid, sodium alginate, dextrin, carbomer, maltodextrin, methylcellulose, ethylcellulose, hydroxyethylcellulose, hydroxypropyl cellulose (HPC), hydroxypropyl methylcellulose (HPMC), hydroxyethyl methylcellulose, carboxymethyl cellulose sodium, cottonseed oil, povidone (PVP), ceratonia, dextrose, polydextrose, starch, gelatin, pregelatinized starch, hydrogenated vegetable oil type I, maltodextrin, microcrystalline cellulose, polyethylene oxide, polymethacrylates and mixtures thereof. Binder can be present in powder form or as a dispersion or mixture of both, in intra and/or extra granular phase of the composition. The amount of binder according to the present invention ranges from 0 to about 50% by weight of the composition. In an embodiment, the binder according to the present invention is present in an amount of about 50% or less, 40% or less, e.g. 30% or less, 20% or less, 10% or less, 5% or less.

Various useful disintegrants and/or super-disintegrants include, but are not limited to, croscarmellose sodium, carboxymethyl cellulose sodium, carboxymethyl cellulose calcium, povidone, crospovidone, polacriilin potassium, sodium starch glycolate, alginic acid, sodium alginate, calcium phosphate, colloidal silicon dioxide, docusate sodium, guar gum, low substituted hydroxypropyl cellulose, magnesium aluminum silicate, methylcellulose, microcrystalline cellulose, silicified microcrystalline cellulose, starch, pre-gelatinized starch and/or combinations thereof. The disintegrant can be present in intra-granular or extra-granular phase or in both (intra and extra granular) phase of the composition. The amount of disintegrant according to the present invention ranges from 0 to about 40% by weight of the composition. In an embodiment, the disintegrant according to the present invention is present in an amount of about 40% or less, 30% or less, e.g. 20% or less, 10% or less. In a preferred embodiment, the disintegrant according to the present invention is present in an amount of about 0.01% to about 20% by weight of the composition.

Pharmaceutically acceptable lubricants include, but are not limited to, stearic acid, zinc stearate, sucrose stearate, sodium benzoate, hydrogenated vegetable oil, calcium stearate, adipic acid, glyceryl palmitostearate, glycerine monostearate, medium-chain triglycerides, glyceryl behenate, sodium lauryl sulphate, sodium stearyl fumarate, magnesium lauryl sulphate, magnesium stearate, polyethylene glycol. The amount of lubricant according to the present invention ranges from 0 to about 20% by weight of the composition. In an embodiment, the lubricant according to the present invention is present in an amount of about 20% or less, e.g. 10% or less, 5% or less. In a preferred embodiment, the lubricant according to the present invention is present in an amount of about 0.01% to about 5% by weight of the composition.

Various useful effervescent agents include, but are not limited to, food acids and acids such as tartaric acid, fumaric acid, citric acid, succinic acid, malic, adipic, sodium bicarbonate, sodium carbonate, potassium carbonate, potassium bicarbonate, magnesium carbonate, and or like compounds. The amount of effervescent agents according to the present invention ranges from 0 to about 90% by weight of the composition. In an embodiment, the effervescent agents according to the present invention are present in an amount of about 90% or less, 80% or less, e.g. 70% or less, 60% or less, 50% or less, 40% or less, 30% or less, 20% or less, 10% or less.

Various useful surfactants include, but are not limited to, sodium lauryl sulphate, polysorbate (e.g. polysorbate 20, polysorbate 40, polysorbate 60, and polysorbate 80), cetrimide, cetyl alcohol, stearyl alcohol, cetyl stearyl alcohol, cholesterol, polyethylene glycols, polyglycerin fatty acid esters such as decaglyceryl monolaurate and decaglyceryl monomyristate, sorbitan fatty acid esters such as sorbitan monostearate, polyoxyethylene sorbitan fatty acid esters such as polyoxyethylene sorbitan monooleate, polyoxyethylene alkyl ether such as polyoxyethylene lauryl ether, polyoxyethylene castor oil, polyoxyethylene polyoxy propylene block copolymers such as poloxamer (such as poloxamer 124, poloxamer 188, poloxamer 237, poloxamer 338, and poloxamer 407), and combinations thereof. The amount of surfactant according to the present invention ranges from 0 to about 30% by weight of the composition. In an embodiment, the surfactant according to the present invention is present in an amount of about 30% or less, e.g. 20% or less, 10% or less, 5% or less, or 2% or less. In a preferred embodiment, the surfactant according to the present invention is present in an amount of about 0.01% to about 5% by weight of the composition.

Suitable pH regulating agents include, but are not limited to, disodium hydrogen phosphate, sodium dihydrogen phosphate and the equivalent potassium salt, alkaline oxides (potassium oxide, sodium oxide, barium oxide, magnesium oxide and aluminium oxide), bases including organic bases, inorganic bases such as sodium hydroxide, potassium hydroxide, calcium hydroxide, calcium oxide, citrate, phosphate, borate salts, organic and inorganic acids such as citric acid, lactic acid, acetic acid, formic acid, oxalic acid, uric acid, malic acid, tartaric acid, succinic acid, benzoic acid, sorbic acid, ascorbic acid, phosphoric acid, boric acid, hydrochloric acid, 1 N hydrochloric acid, sulphuric acid, nitric acid, phosphoric acid, hydrobromic acid, hydroiodic acid or hydrofluoric acid, as known in the art such as those described above, carbonate salts (sodium carbonate, potassium carbonate, calcium carbonate, ammonium carbonate, or magnesium carbonate), bicarbonate salts (sodium bicarbonate, potassium bicarbonate, calcium bicarbonate, ammonium bicarbonate, or magnesium bicarbonate), amino acids (arginine, histidine, lysine, glycine), and amino sugars (N-acetylglucosamine, galactosamine, glucosamine, sialic acid, or meglumine). Binary buffer systems are also included in the ambit of the present invention. "pH regulating agent" shall be defined as a compound that, alters or adjusts the pH of the local environment. In the context of the present invention, a "pH regulating agent" alters or adjusts the pH of the sublingual area upon dissolving. The pH of the composition as mentioned in the present invention also refers to the pH of the sublingual area upon dissolving and/or pH of the drug in solution. The amount of pH regulating agents according to the present invention ranges from 0 to about 20% by weight of the composition. In an embodiment, the pH regulating agents according to the present invention are present in an amount of about 20% or less, e.g. 10% or less, 5% or less, 3% or less.

Suitable glidants include, but are not limited to, calcium silicate, magnesium silicate, magnesium trisilicate, stearic acid and its derivatives or esters like magnesium stearate, calcium stearate, and sodium stearate and the corresponding esters such as sodium stearyl fumarate, talc, colloidal silicon dioxide, tribasic calcium phosphate, starch, starch derivatives or mixtures thereof. The amount of glidant according to the present invention ranges from 0 to about 20% by weight of the composition. In an embodiment, the glidant according to the present invention is present in an amount of about 20% or less, e.g. 10% or less, 5% or less, 3% or less. In a preferred embodiment, the glidant according to the present invention is present in an amount of about 0.01% to about 5% by weight of the composition.

Various useful salivating agents include, but are not limited to, alkyl aryl sulfonates, alkyl sulfonates, alkyl sulfates, sulfonated amides and amities, sulfated and sulfonated esters and ethers, mono-, di-, and triglycerides, diacetyl tartaric esters of monoglycerides, polyglycerol esters, sorbitan esters and ethoxylates, lactylated esters, phospholipids such as lecithin, polyethoxylated esters, polyoxyethylene sorbitan esters, propylene glycol esters, sucrose esters, citric acid, malic acid, tartrate, sodium chloride, potassium chloride, and mixtures thereof. The amount of salivating agents according to the present invention ranges from 0 to about 30% by weight of the composition. In an embodiment, the salivating agents according to the present invention are present in an amount of about 30% or less, e.g. 20% or less, 10% or less, 5% or less, or 2% or less.

Various useful solubilizing agents, permeation/penetration enhancers include, but are not limited to, alcohols, polyethylene glycols, cyclodextrins (cyclodextrin analogs, such as but not limited to, alpha-, beta-, and gamma-cyclodextrin analogs and their derivatives such as sulfobutylether-β-cyclodextrin (SBECD)) or betadex sulfobutyl ether sodium, sodium lauryl sulfate, sodium carboxymethyl cellulose, povidone, sucrose laurate or sucrose oleate, sodium dodecyl sulfate (SDS) or palmitoyl carnitine chloride (PCC) and citric acid. The amount of solubilizing agents, permeation/penetration enhancers according to the present invention ranges from 0 to about 50% by weight of the composition. In an embodiment, the solubilizing agent, permeation enhancer according to the present invention is present in an amount of about 50% or less, 40% or less, 30% or less, 20% or less, 10% or less, e.g. 5% or less, 3% or less, 2% or less, or 1% or less. In a preferred embodiment, the solubilizing agent according to the present invention is present in an amount of about 0.01% to about 35% by weight of the composition.

Various useful preservatives include, but are not limited to, citric acid, butylated hydroxyanisole, vitamin C, vitamin E, parabens (methylparaben, propylparaben), phenyl ethyl alcohol, sorbic acid, benzyl alcohol, alkyl-benzyl dimethylammonium chloride with a chain length of from C₈ to C₁₈ in the alkyl moiety, m-cresol or alkyl-4-hydroxybenzoate. The amount of preservatives according to the present invention ranges from 0 to about 20% by weight of the composition. In an embodiment, the preservatives according to the present invention are present in an amount of about 20% or less, e.g. 10% or less, 5% or less, 3% or less.

The term "taste masking agents" or "taste masker", when used herein, refers to taste receptor blockers, compounds that mask the chalkiness, grittiness, dryness, and/or astringent or bitter taste properties of an active compound. Taste masking agents are selected from Opadry AMB, sodium starch glycolate, bitter taste masker flavors, commercially available bitter masker, carbopol polymers, PEG-5M, sodium acetate, ethylcellulose, cyclodextrin, beta-cyclodextrin, polyvinyl acetate dispersion, trehalose, vinyl acetate, polystyrene, cellulose acetate butyrate, methacrylic acid, and methyl methacrylates such as Eudragit(R) L100, polymethacrylates (such as Eudragit(R) L100), sodium chloride, polyethoxylated castor oil, Kolliphor® RH 40, ion-exchange resins such as Amberlite, Amberlite® CG 50, Amberlite® IRP-64, Amberlite® IRP-69, Indion®204, Indion® 214, Indion® 234, Indion® CRP 244, Indion® CRP 254, Carbomer 934, Carbomer 974, Carbomer 971, Carbopol® 934P NF, Carbopol® 971P, Carbopol® 974P NF, Sentry® Polyox® WSR N80 NF, natural or synthetic fatty type or other flavorant such as cocoa, chocolate (especially mint chocolate), cocoa butter, milk fractions, vanillin butterfat, egg or egg white, peppermint oil, wintergreen oil, spearmint oil, and similar oils. Compounds which reduce throat catch include for example high solubility acids include amino acids (e.g. alanine, arginine, etc.), glutaric, ascorbic, malic, oxalic, tartaric, malonic, acetic, citric acids, low solubility acids include oleic, stearic, and aspartic acids plus certain amino acids such as glutamic acid, glutamine, histidine, isoleucine, leucine, methionine, phenylalanine, serine, tryptophan, tyrosine, valine, and fumaric acid. In an embodiment, the taste masking agents, according to the present invention is present in an amount of about 50% or less, 40% or less, 30% or less, 20% or less, 10% or less, e.g. 5% or less, 3% or less, 2% or less, or 1% or less. In an embodiment, the amount of the taste masking agent is from about 0.01% to about 10% of the composition.

Various useful flavoring agents and/or mouth-feel improvers include, but are not limited to, mint powder, menthol, vanillin, aspartame, acesulfame potassium, saccharin sodium, aromatics and/or natural oils, synthetic flavor oils, extracts from plants, leaves, flowers, fruits and combinations thereof, commercially available orange, grape, cherry and bubble gum flavors, tutti-frutti flavors, bitter taste masker flavors, commercially available bitter masker flavor, peppermint flavour, and mixtures thereof. The amount of flavoring agents according to the present invention ranges from 0 to about 10% by weight of the composition. In an embodiment, the flavoring agents according to the present invention are present in an amount of about 10% or less, e.g. 5% or less, 3% or less, 2% or less, or 1% or less. In a preferred embodiment, the flavoring agent according to the present invention is present in an amount of about 0.01% to about 10% by weight of the composition.

Various useful sweetening agents or sweeteners include, but are not limited to, sugars such as sucrose, sucralose, glucose, dextrose, maltose, fructose, artificial sweeteners (such as saccharin, saccharin sodium, aspartame, acesulfame, acesulfame potassium, neohesperidine dihydrochalcone, mono-ammonium glycyrrhizinate, sugar alcohols (such as mannitol, xylitol, lactitol, maltitol syrup), thaumatin, monellin, dihydrochalcones, dipotassium glycyrrhizinate, stevia and mixtures thereof, present conveniently in an amount of from 0 to about 65% by weight of the composition. In an embodiment, the sweetening agents according to the present invention is in an amount of about 65% or less, e.g. 60% or less, 50% or less, 40% or less, 30% or less, 20% or less, 10% or less, 5% or less, 3% or less, 2% or less, or 1% or less. In a preferred embodiment, the sweetening agent according to the present invention is present in an amount of about 0.01% to about 10% by weight of the composition.

Various other excipients or inert ingredients such as antioxidants, acidulants, effervescent agents, water-soluble and/or water-dispersible carrier materials, bio/mucoadhesion promoting agents, stabilizers, emulsifiers, plasticizers, suspending agents, and coloring agents are also covered under the ambit of the present invention. The amount of these excipients according to the present invention may range from 0 to about 90% by weight of the composition. In an embodiment, these excipients according to the present invention are present in an amount of about 90% or less, 80% or less, e.g. 70% or less, 60% or less, 50% or less, 40% or less, 30% or less, 20% or less, 10% or less.

The invention is further defined by reference to the following examples describing in detail the methods for the preparation and testing of pharmaceutical compositions. The following examples are provided to illustrate embodiments of the disclosure but they are by no means intended to limit its scope.

**EXAMPLES:** Sublingual compositions were prepared by using quantitative formula as given in Tables 1-4 (Quantity/Tablet (%w/w)).

**Table 1: Examples (Ex.) 1-3:**

| **S. No.** | **Ingredient** | **Function** | **Ex. 1** | **Ex. 2** | **Ex. 3** |
|---|---|---|---|---|---|
| 1. | Antiviral Drug / Remdesivir | Drug | 0.1-97 | 5-85 | 0.1-89 |
| 2. | Mannitol / Sorbitol / Xylitol / Microcrystalline Cellulose | Diluent | 0-70 | 30-60 | 10-70 |
| 3. | HPMC / HPC / Povidone | Binder | 0-30 | 0-20 | 0.01-20 |
| 4. | Sodium starch glycolate / Croscarmellose sodium / PGS / Crospovidone | Disintegrant | 0-20 | 0-10 | 0.01-15 |
| 5. | Gelatin / Sodium alginate | Carrier | 0-50 | 0-30 | - |
| 6. | Sodium Lauryl Sulphate / Polysorbate 80 / Poloxamer | Surfactant | 0-5 | 0-2 | - |
| 7. | Citric acid | pH adjusting / salivating agent / solubilizer | 0-5 | 0-5 | 0.01-15 |
| 8. | Beta-Cyclodextrin | Solubilizing agent | - | - | 0.01-40 |
| 9. | Talc / Colloidal silicon dioxide | Glidant | 0-5 | 0-2 | 0.01-3 |
| 10. | Magnesium Stearate | Lubricant | 0.1-5 | 0.1-2 | 0.01-3 |
| 11. | Butylated Hydroxyanisole | Preservative / Antioxidant | 0-5 | 0.1-2 | - |
| 12. | Sucralose / Saccharin Sodium / Aspartame / Acesulfame Potassium | Sweetener | 0.1-5 | 0.1-2 | 0.01-5 |
| 13 | Milk Protein Concentrate | Bio / Mucoadhesive | 0-20 | 0-10 | 0-20 |
| 14. | Orange Flavour (optional) | Flavoring agent | - | - | 0-5 |
| 15. | Aqueous or Alcoholic or Acidic solvent or any mixture thereof | Solvent | q.s. | q.s. | q.s. |

| | | | | | |
|---|---|---|---|---|---|
| **Procedure:** The compositions according to Table 1 were prepared by wet granulation. | | | | | |

**Table 2: Examples 4-9:**

| **S. No.** | **Ingredient** | **Function** | **Ex. 4** | **Ex. 5** | **Ex. 6** | **Ex. 7** | **Ex. 8** | **Ex. 9** |
|---|---|---|---|---|---|---|---|---|
| 1. | Remdesivir | Drug | 0.1-30 | 10.5 | 10 | - | 30 | - |
| 2. | GS-441524 | Drug | - | - | - | 0.1-30 | - | - |
| 3. | Molnupiravir | Drug | - | - | - | - | - | 30 |
| 4. | Microcrystall ine Cellulose | Diluent | 20-70 | 34.8 | 33.1 | 20-70 | 21.9 | 21.9 |
| 5. | Povidone | Binder | 0.1-5 | 0.5 | 0.5 | 0.1-5 | 0.5 | 0.5 |
| 6. | Crospovidone | Disintegrant | 0.01-10 | 6.3 | 6 | 0.01-10 | 6.3 | 6.3 |
| 7. | Citric acid | pH regulating agent | 0.01-8 | 7.9 | 7.5 | 0.01-8 | 7.9 | 7.9 |
| 8. | Beta-Cyclodextrin | Solubilizing agent | 0.01-35 | 31.6 | 35 | 0.01-35 | 25 | 25 |
| 9. | Colloidal silicon di-oxide | Glidant | 0.01-3 | 2.4 | 2.2 | 0.01-3 | 2.4 | 2.4 |
| 10. | Magnesium Stearate | Lubricant | 0.01-2 | 1.8 | 1.7 | 0.01-2 | 1.8 | 1.8 |
| 11. | Acesulfame Potassium | Sweetener | 0.01-3 | 2.6 | 2.5 | 0.01-3 | 2.6 | 2.6 |
| 12. | Orange Flavor | Flavoring agent | 0.01-3 | 1.6 | 1.5 | 0.01-3 | 1.6 | 1.6 |
| 13. | Ethanol | Solvent | q.s. | q.s. | q.s. | q.s. | q.s. | q.s. |
| 14. | 1 N HCl | Solvent / pH regulating agent | q.s. | q.s. | q.s. | q.s. | q.s. | q.s. |

| | | | | | | | | |
|---|---|---|---|---|---|---|---|---|
| **Procedure** - i) Diluent, disintegrant, glidant, and binder were sifted through a suitable sieve, ii) drug and solubilizing agent were dispersed into a suitable solvent, iii) step i) blend was granulated using the dispersion of step ii), iv) wet granules were dried followed by passing the dried granules through a suitable sieve, v) dried granules were blended with suitable excipients such as glidant, pH regulating agent, sweetener, flavor (optional), solubilizing agent (optional) and lubricant and were sifted through a suitable sieve, and vi) the blend of step v) was compressed into tablets using suitable punches. | | | | | | | | |

**Table 3: Examples 10-14:**

| **S. No.** | **Ingredient** | **Function** | **Ex. 10** | **Ex. 11** | **Ex. 12** | **Ex. 13** | **Ex. 14** |
|---|---|---|---|---|---|---|---|
| 1. | Remdesivir | Drug | 20 | 13.3 | 0.7 | 6.7 | 33.3 |
| 2. | Mannitol | Diluent | 63.8 | 28.7 | 28.7 | 28.7 | 28.7 |
| 3. | Microcrystalline cellulose | Diluent | - | 26.4 | 39.1 | 33.1 | 6.4 |
| 4. | Beta Cyclodextrin | Solubilizing | 2 | 13.3 | 13.3 | 13.3 | 13.3 |
| | | agent | | | | | |
| 5. | Povidone | Binder | 1 | 0.7 | 0.7 | 0.7 | 0.7 |
| 6. | Crospovidone | Disintegrant | 4 | 8 | 8 | 8 | 8 |
| 7. | Citric acid | pH regulating agent | 5 | - | - | - | - |
| 8. | Colloidal silicon dioxide | Glidant | 1.5 | 3 | 3 | 3 | 3 |
| 9. | Magnesium Stearate | Lubricant | 2.3 | 2.3 | 2.3 | 2.3 | 2.3 |
| 10. | Acesulfame Potassium | Sweetener | 0.5 | 3.3 | 3.3 | 3.3 | 3.3 |
| 11. | Peppermint flavor | Flavoring agent | - | 1 | 1 | 1 | 1 |
| 12. | Ethanol | Solvent | q.s. | q.s. | q.s. | q.s. | q.s. |
| 13. | Water | Solvent | q.s. | - | - | - | - |
| 14. | 1 N HCl | Solvent / pH regulating agent | - | q.s. | q.s. | q.s. | q.s. |

| | | | | | | | |
|---|---|---|---|---|---|---|---|
| **Procedure - Table 3:** i) One or more diluents, disintegrant, binder and glidant were sifted through a suitable sieve, ii) solubilizing agent was dissolved in a suitable solvent followed by the addition of another suitable solvent, iii) drug was dissolved in solution as prepared in step ii), followed by pH adjustment with IN hydrochloric acid at suitable pH (such as between pH 2 to pH 4), iv) step i) blend was granulated using the solution of step iii), v) wet mass was sifted through a suitable sieve, vi) wet granules were dried and sifted through a suitable sieve, vii) dried granules were blended with suitable disintegrant, glidant, sweetener, flavor (optional) and lubricant and sifted through a suitable sieve and added to step vi) granules, and viii) the blend of step vii) was compressed into tablets using suitable punches. | | | | | | | |

**Table 4: Examples 15-17: (Quantity/Tablet (%w/w))**

| **S. No.** | **Ingredient** | **Function** | **Ex. 15** | **Ex. 16** | **Ex. 17** |
|---|---|---|---|---|---|
| 1 | Microcrystalline Cellulose | Diluent | 50 | 47 | 47 |
| 2 | Crospovidone | Disintegrant | 6.5 | 6.6 | 6.6 |
| 3 | Remdesivir | Drug | 6.7 | - | - |
| 4 | Molnupiravir | Drug | - | 10 | - |
| 5 | GS-441524 | Drug | - | - | 10 |
| 6 | Povidone | Binder | 0-0.5 | 0-1 | 0-1 |
| 7 | Beta Cyclodextrin | Solubilizing agent | 26.6 | 26.6 | 26.6 |
| 8 | Purified Water, Ethanol | Solvent | q.s. | q.s. | q.s. |
| 9 | Hydrochloric Acid, Sodium | pH regulating agent | q.s. | q.s. | q.s. |
| | Hydroxide | | | | |
| 10 | Thaumatin, Taste Masker flavor, Peppermint flavor | Sweetener, Taste masking agent, Flavoring agent | 8.2 | 8.0 | 8.0 |
| 11 | Colloidal silicon di-oxide | Glidant | 1.0 | 0.9 | 0.9 |
| 12 | Magnesium Stearate | Lubricant | 1.0 | 0.9 | 0.9 |

| | | | | | |
|---|---|---|---|---|---|
| **Procedure:** i) Diluent and disintegrant were sifted through a suitable sieve, ii) solubilizing agent was dissolved in a suitable solvent, followed by addition of drug (remdesivir/molnupiravir/GS-441524), followed by addition of IN hydrochloric acid and followed by addition of IN Sodium Hydroxide solution to adjust the suitable pH of the solution (such as between pH 2 to pH 7.0), iii) step i) blend was granulated using a solution of step ii), iv) wet granules were dried followed by passing the dried granules through a suitable sieve, v) dried granules were blended with suitable excipients such as diluent, disintegrant, sweetener, taste masking agent, flavoring agent, glidant, and lubricant and were sifted through a suitable sieve, and vi) the blend of step v) was compressed into tablets using suitable punches. | | | | | |

**Results: Dissolution:** The dissolution profile of sublingual tablet compositions prepared using quantitative compositions (Examples 5, 11-14), as given below in Table 5 was measured in 900 ml of 0. IN Hydrochloric acid using a USP II apparatus (Paddle) at a temperature of 37±0.5°C and a rotation speed of 50 revolutions per minute. The quantitative composition as given below in Table 5 exhibited at least 80% of drug release in 15 minutes or less, accordingly the dissolution profiles of all of the formulations were found to be acceptable.

The inventors determined that while certain excipients are critical for a tablet to function as a sublingual tablet, other excipients are necessary for patient acceptance of a formulation as a sublingual tablet. For example, if the tablet is overly bitter, causes numbness, or otherwise has organoleptic properties that prevent an individual from retaining the tablet under the tongue for a sufficient amount of time to permit dissolution, the tablet will not be effective. The inventors have determined that by including a combination of a sweetener, a taste masking agent, and a flavoring agent, the composition can have the suitable organoleptic properties necessary for a sublingual tablet. For example, the inventors determined that a combination of thaumatin, a mixture of conventional taste masking agents, and a flavor such as peppermint will provide the necessary organoleptic properties for a patient to accept leaving the sublingual tablet in the mouth for a sufficient amount of time.

A combination of other excipients are necessary to achieve the release and drug dissolution for a sublingual tablet. A combination of the solubilizing agent, diluent, disintegrant, and pH regulating agent are necessary for proper dissolution of the tablet to provide the desired sublingual absorption. The addition of a glidant and/or lubricant ensure the manufacturability of the tablet. While it is known to provide conventional rapid disintegrating tablets, that knowledge does not necessarily result in suitable drug dissolution for sublingual administration. Instead, the drug leaves the mouth, passes through the throat, and enters the gastrointestinal tract where it is absorbed. But this is well known in the art to be different from sublingual administration and adsorption. The inventors have determined that for the sublingual administration of an antiviral active ingredient, it is necessary to use the excipients described herein in the combinations and ratios disclosed.

**Table 5 - Percent Drug Release of Various Compositions**

| **Time Point (min.)** | **% Drug release** | | | | |
|---|---|---|---|---|---|
| | **Ex. 5** | **Ex. 11** | **Ex. 12** | **Ex. 13** | **Ex. 14** |
| **5** | **80** | **87** | **67** | **61** | **57** |
| **10** | **87** | **93** | **90** | **87** | **80** |
| **15** | **89** | **95** | **88** | **91** | **89** |
| **30** | **91** | **95** | **89** | **92** | **94** |

In developing the dissolution testing procedure the inventors examined the effect of pH on dissolution to ensure that the dissolution media used is discerning enough to predict whether a formulation will be suitable as a sublingual tablet. The inventors prepared three reference formulations of remdesivir to analyze the dissolution profile of the compositions in three different dissolution media: (a) pH 1.2, 0.1 N HCl/USP-II/900 ml/50 RPM; (b) pH 3.0, Mcllvaine Buffer/USP-I/900 mL/100 RPM; and (c) pH 6.8 Phosphate Buffer/USP-11/900 mL/50 RPM. By varying the pH of the dissolution media, the inventors developed a more discerning method to differentiate between dissolution media for testing in vitro dissolution.

**Table 6 - Dissolution Testing Reference Examples**

| | | **Ref. Example A** | | **Ref. Example B** | | **Ref. Example C** | |
|---|---|---|---|---|---|---|---|
| **Ingredient** | **Category** | mg/tab | %w/w | mg/ta b | %w/w | mg/tab | % w/w |
| | Intragra nular | | | | | | |
| Remdesivir | Active | 0.750 | 0.25 | 30.00 | 10.00 | 15.39 | 5.13 |
| MCC | Filler | 180.00 | 60.00 | 192.0 0 | 64.00 | 195.00 | 65.00 |
| Povidone | Binder | 3.00 | 1.00 | 15.00 | 5.00 | 9.00 | 3.00 |
| Crospovidone | Disintegrant | 3.00 | 1.00 | 30.00 | 10.00 | 16.50 | 5.50 |
| Citric Acid | Taste enhancing agent | 0.00 | 0.00 | 0.00 | 0.00 | 16.50 | 5.50 |
| Citric Acid | Stabilizer | 1.50 | 0.50 | 30.00 | 10.00 | 0.00 | 0.00 |
| Tartaric Acid | Stabilizer | 0.00 | 0.00 | 0.00 | 0.00 | 15.75 | 5.25 |
| | Granulating Solution | | | | | | |
| Purified water | Granulating solvent | q.s. | q.s. | q.s. | q.s. | q.s. | q.s. |
| Ethyl alcohol | Granulating solvent | q.s. | q.s. | q.s. | q.s. | q.s. | q.s. |
| | Extragranular | | | | | | |
| MCC | Filler | 108.75 | 36.25 | 0.000 | 0.00 | 28.86 | 9.62 |
| Magnesium stearate | Lubricant | 3.00 | 1.00 | 3.000 | 1.00 | 3.00 | 1.00 |
| | Total | 300.00 | 100.00 | 300.0 0 | 100.00 | 300.00 | 100.00 |

| | | | | | | | |
|---|---|---|---|---|---|---|---|
| **Procedure:** i) Drug, diluent, binder, disintegrant, taste enhancing agent/stabilizer were sifted through a suitable sieve, ii) step i) blend was granulated using a solution of granulating solvents, iii) wet granules were dried followed by passing the dried granules through a suitable sieve, iv) dried granules were blended with suitable excipients such as diluent, and lubricant and were sifted through a suitable sieve, and v) the blend of step iv) was compressed into tablets using suitable punches. | | | | | | | |

**Table 7 - Dissolution Media 0.1N HCl/USP-II/900 mL/50 RPM (pH 1.2)**

| **% Dissolution** | **Reference Ex A** | **Reference Ex B** | **Reference Ex C** | **Ex 15** |
|---|---|---|---|---|
| **Time (min.)** | **% Drug release** | | | |
| **5** | 69 | 86 | 83 | 63 |
| **10** | 77 | 88 | 86 | 88 |
| **15** | 77 | 88 | 89 | 89 |
| **20** | 79 | 88 | 89 | 90 |
| **30** | 80 | 89 | 90 | 90 |
| **Inf.** | 82 | 95 | 92 | 94 |

**Table 8 - Dissolution Media Mcllvaine Buffer/USP-I/900 mL/100 RPM (pH 3.0)**

| **% Dissolution** | **Reference Ex A** | **Reference Ex B** | **Reference Ex C** | **Ex 15** |
|---|---|---|---|---|
| **Time (min.)** | **% Drug release** | | | |
| **5** | 42 | 30 | 47 | 98 |
| **10** | 53 | 35 | 53 | 99 |
| **15** | 57 | 38 | 58 | 99 |
| **20** | 60 | 41 | 60 | 99 |
| **30** | 65 | 44 | 64 | 99 |
| **Inf.** | 72 | 60 | 73 | 99 |

**Table 9 - Dissolution Media Phosphate Buffer/USP-II/900 mL/50 RPM (pH 6.8)**

| **% Dissolution** | **Reference Ex A** | **Reference Ex B** | **Reference Ex C** | **Ex 15** |
|---|---|---|---|---|
| **Time (min.)** | **% Drug release** | | | |
| **5** | 43 | 22 | 34 | 44 |
| **10** | 49 | 25 | 40 | 78 |
| **15** | 53 | 28 | 42 | 84 |
| **20** | 55 | 30 | 44 | 86 |
| **30** | 58 | 32 | 47 | 86 |
| **Inf.** | 68 | 36 | 53 | 98 |

The data reported in Table 7 demonstrates that the three Reference examples and Example 15 each have at least 75% dissolution within 15 to 30 minutes when using 0.1N HC1/USP-II/900 mL/50 RPM (pH 1.2), thereby meeting the dissolution requirements for a sublingual tablet. However, because it is well known that in a highly acidic environment the active ingredient has increased solubility, the inventors also tested dissolution using a Mcllvaine Buffer/USP-I/900 mL/100 RPM. This dissolution media has a pH of 3.0. The data reported in Table 8 demonstrates that the three Reference examples fail to attain the required at least 75% dissolution within 30 minutes in the McIlvaine media. In contrast, Example 15 attained the required at least 75% dissolution within 30 minutes, thereby meeting the dissolution requirements for a sublingual tablet. To further refine the in vitro dissolution testing method, the inventors also tested a phosphate buffer/USP-11/900 ml/50 RPM dissolution system. This dissolution media has a pH of 6.8, which is a good model for the physiological pH of the saliva in the human mouth. The data reported in Table 9 demonstrates that the three Reference examples fail to attain the required at least 75% dissolution within 30 minutes while Example 15 has at least 75% dissolution within 30 minutes, thereby meeting the dissolution requirements for a sublingual tablet.

Table 10 provides a summary of the dissolution profile of the sublingual tablet composition prepared in Example 15 measured in 900 ml of 0.1N HCl (pH 1.2) using a USP-II apparatus; 900 ml of McIlvaine Buffer (pH 3.0), using a USP I apparatus (basket) at a temperature of 37±0.5°C and a rotation speed of 100 revolutions per minute and 900 ml of Phosphate buffer (pH 6.8), using a USP II apparatus (paddle) at a temperature of 37±0.5°C and a rotation speed of 50 revolutions per minute. The quantitative composition as given below in Table 10 exhibited at least 75% of drug release in 15 minutes or less, accordingly the dissolution profiles of all of the formulation was found to be acceptable.

**Table 10 - Percent Drug Release of the Composition of Example 15**

| **Time Point (min.)** | **% Drug release** | | |
|---|---|---|---|
| | 0.1N HCl (pH 1.2) | McIlvaine Buffer (pH 3.0) | Phosphate Buffer (pH 6.8) |
| **5** | 63 | 98 | 44 |
| **10** | 88 | 99 | 78 |
| **15** | 89 | 99 | 84 |
| **20** | 90 | 99 | 86 |
| **30** | 90 | 99 | 86 |

The inventors have determined that for absorption of the drug from a sublingual dosage form, at least 75% dissolution of the active ingredient within 15 minutes should be attained in both (a) Mcllvaine Buffer/USP-I/900 mL/100 RPM (pH of 3.0) and (b) phosphate buffer/USP-11/900 ml/50 RPM dissolution system (pH of 6.8). Example 15 reached this in vitro threshold and therefore in vivo animal and human testing was conducted, as described in more detail below. In contrast, because Reference Examples A-C did not attain the required dissolution, they were not suitable for a sublingual dosage form.

The pH results for Examples 5, 6, and 10-15 are provided below in Table 11.

**Table 11. pH Results:**

| **Example No.** | **5** | **6** | **10** | **11** | **12** | **13** | **14** | **15** |
|---|---|---|---|---|---|---|---|---|
| **pH** | 3.7 | 3.7 | 3.7 | 3.8 | 2-6.5 | | | 3.4 |

The measurement of related substances for Examples 10-15 is provided in Table 12.

**Table 12. Related Substance:**

| **Example No.** | **10** | **11** | **12** | **13** | **14** | **15** |
|---|---|---|---|---|---|---|
| **Related Substance** (as measured by HPLC) | Less than 2% | | | | | |

| | | | | | | |
|---|---|---|---|---|---|---|
| Additional measurements of the physical characteristics of the tablets of Examples 10-11 and 15 are provided in Table 13. | | | | | | |

**Table 13 Other Results:**

| **Example No.** | **10** | **11** | **15** |
|---|---|---|---|
| **Hardness** | 18-20 | 22-25 | 29-36 |
| **Friability** | NMT 1% | | |
| **Particle Size** | D₉₀ less than about 200 µm | | |
| **Disintegration Time (seconds)** | 20-30 | 45-50 | 60-116 |
| **Assay (%)** | 90.0-110.0 | | |

### Stability:

The sublingual tablet dosage form prepared in Example 15 was subjected to Accelerated stability testing as per the ICH guidelines at temperature/relative humidity of 40°±2° C/75%±5% RH for 2 months. The sublingual tablet dosage form was placed in an Aluminum blister and analyzed for drug content by High-Performance Liquid Chromatography (HPLC) method. As reported in Table 14, the dosage form of Example 15 was found to be stable and exhibited the following assay values:

**Table 14: Stability**

| **Study Period** | **Acceptable limits** | **Assay Results** |
|---|---|---|
| Initial | 90% - 110% | 100.9% |
| After one month | 90% - 110% | 98.4% |
| After two months | 90% - 110% | 98.7% |

### Example 16 - Pharmacokinetic Studies in Dogs:

The remdesivir sublingual tablets formulation of Example 15 was evaluated for its pharmacokinetic in dogs. The purpose of this study was to determine the pharmacokinetic profiles of remdesivir sublingual tablets in dogs and to assess the absorption characteristics/pharmacokinetic exposure of the predominantly circulating nucleoside metabolite (GS-441524) of remdesivir from remdesivir sublingual tablets and compare it with remdesivir for injection.

This preclinical study was conducted in male Beagle Dogs. Six dogs of *Canis lupus familiaris* (beagle dog) of weight range from 10.05 to 11.25 kg, were injected with remdesivir for injection 60 mg (12 mL of 5 mg/mL) intravenous infusion of remdesivir as control and six dogs received remdesivir sublingual tablet (20 mg X 3 sublingual tablets).

**Dosing Procedure: Sublingual Tablet (Test):** Test formulations were administered to dogs via sublingual route. **Remdesivir IV infusion (Reference):** Reference formulations were administered to dogs via IV infusion. The flow rate of the infusion was kept as 6 mL/hour.

**Sampling Schedule:** The blood sample was collected at various time points from 0 to 96 hours post dose. At specific time points, approximately 1 mL of blood was collected from each dog.

**Primary PK Parameters:** Cₘₐₓ, AUC₀₋ₜ, and AUC_{0-inf} of the predominantly circulating nucleoside metabolite (GS-441524) obtained with remdesivir sublingual tablets as compared to remdesivir injection.

**Pharmacokinetic and Statistical Analysis:** Pharmacokinetic parameters were determined using Phoenix® WinNonlin® 8.3 Software. Statistical evaluation was done by using the statistical software package SAS® for Windows, version 9.4, SAS Enterprise Guide 6.1 (Statistical Analysis System, SAS-Institute, Cary NC, USA).

**Table 15: Pharmacokinetic Parameters of Example 15 in Dog Study**

| **Parameters** | **Cₘₐₓ [ng/mL]** | **AUC₀₋ₜ [ng*hr/mL]** | **AUC_{0-inf} [ng*hr/mL]** |
|---|---|---|---|
| **Geometric Mean (T)** | **623.4** | **4424.7** | **4473.7** |
| **Geometric Mean (R)** | **449.6** | **6342.4** | **6513.3** |
| **Point Estimate (T/R Ratio) (%)** | **138.7** | **69.8** | **68.7** |

**Conclusion:** Predominant circulating metabolite of remdesivir GS-441524 could be detected from 0.33 hours after dose administration till 96 hours post-dose. The peak concentration of the nucleoside metabolite was observed to be higher with sublingual tablets as compared to intravenous (IV) remdesivir (Cₘₐₓ T/R ratio: ∼139%). Relative exposure (sublingual/IV injection) to remdesivir metabolite (GS-441524) was ∼70% based on AUCₜ data and ∼69% based on AUC_{inf} parameter. Remdesivir sublingual tablets administered to the beagle dogs were well tolerated by all the animals. No safety/tolerability concerns were identified during this preclinical study. The drug was able to undergo absorption when administered by the sublingual route. It appears to be rapidly converted to its metabolite form (GS-441524). Good exposure to remdesivir metabolite (GS-441524) was obtained. These findings confirm the effective drug delivery via a sublingual route.

### Example 17 - Single-Dose Comparative Pharmacokinetic Exposure Study in Healthy Human Subjects:

The remdesivir sublingual tablets formulation of Example 15 was evaluated for pharmacokinetic parameters in human subjects.
**Objective:** The objective of the trial was to characterize the pharmacokinetic exposure and to assess the steady-state comparison of the test formulation (T) [remdesivir sublingual tablet 20 mg (dose 200 mg/day)] with reference formulation (R) [Jubi-R™
(remdesivir for injection 100 mg) (loading dose of 200 mg/day and maintenance dose of 100 mg/day)] in a healthy adult, human subjects, under fasting conditions. The safety of the study subjects was also monitored and evaluated.
**Study Design:** A comparative pharmacokinetic exposure study was conducted in 24 healthy human subjects. Twelve subjects received remdesivir sublingual tablets and 12 subjects received an intravenous infusion of remdesivir (JUBI-R™) as reference. The study was a randomized, open-label, balanced, two-treatment, one-sequence, one-period, multiple-dose, parallel design, steady-state comparison study of the test product (T) [remdesivir sublingual tablets 20 mg (dose 200 mg/day)] with the reference product (R) [JUBI-R™ (remdesivir for injection 100 mg) (loading dose 200 mg/day, maintenance dose 100 mg/day)] in a healthy adult, human subjects, under fasting conditions.
Healthy, adult human volunteers who were willing to participate in the study and fulfill the inclusion and exclusion criteria were selected for the study. Volunteers aged from 18-45 years with a body weight 50-80 Kg in case of male and/or 45-75 Kg in case of female with body mass index (BMI) in the range of 18.50-24.90 Kg/m² (both inclusive, calculated as weight in kg/height in meter) were selected for the study. The patients were healthy as determined by medical history, clinical and laboratory examination was performed within 21 days before admission for the study.
**Dosing Procedure:** Sublingual tablet (test): 5 sublingual tablets of 20 mg administered over a period of 45 minutes. IV infusion (reference): Dose of 100 mg administered as an intravenous infusion over a period of 120 minutes. The flow rate of the infusion was kept as 2.08 mL/min. Infusion time was 120 minutes.
**Sampling Schedule:** The blood sample was collected at various time points from 0 to 96 hours post dose. At each time point, sample was collected in pre-labeled K2-EDTA vacutainers.
**Primary PK Parameters:** Primary PK parameters were Cₘₐₓ, AUC₀₋ₜ, and AUC_{0-inf} for nucleoside metabolite (GS-441524).
**Evaluation Criteria:** Ratio of geometric least-square means of the test (T) and reference (R) formulations calculated and reported in percentage for Ln-transformed Cₘₐₓ, AUC₀₋ₜ, and AUC_{0-inf} for the predominantly circulating nucleoside metabolite (GS-441524).

### Pharmacokinetic Analysis:

Remdesivir sublingual tablets administered to the healthy humans were well tolerated by all the subjects and no safety and/or tolerability concerns were identified during this study.

**Table 16: Pharmacokinetic Parameters of Example 15 in Humans**

| **Parameter** | **Cₘₐₓ [ng/mL]** | **AUC₀₋ₜ [ng*hr/mL]** | **AUC_{0-inf} [ng*hr/mL]** |
|---|---|---|---|
| **Geometric Mean (T)** | 152.1 | 1593.2 | 1644.3 |
| **Geometric Mean (R)** | 94.0 | 2778.3 | 3030.3 |
| **Point Estimate (T/R Ratio) (%)** | **161.8** | **57.3** | **54.3** |

**Conclusion:** These findings confirm that the drug was able to undergo absorption when administered by the sublingual route in human subjects. It rapidly converted to its metabolite form (GS-441524). Good exposure to metabolite (GS-441524) was obtained. Remdesivir sublingual tablets were well tolerated by all the subjects with no safety and/or tolerability concerns being identified.

### Example 17 - Comparative Multiple-Dose Pharmacokinetic Exposure Study in Healthy Human Subjects:

The remdesivir sublingual tablets formulation of Example 15 was evaluated for pharmacokinetics in human subjects.
**Objective:** A trial to compare remdesivir sublingual tablets to remdesivir injection in healthy adult human subjects. The objective of the trial was to characterize the pharmacokinetic exposure and to assess the steady-state comparison of the test formulation (T) [remdesivir sublingual tablet 20 mg (Dose 200 mg/day)] with reference formulation (R) [Jubi-R™ (remdesivir for injection 100 mg) (loading Dose 200 mg/day, maintenance Dose 100 mg/day) in a healthy adult, human subjects, under fasting conditions. The safety of the study subjects was also monitored and evaluated.
**Study Design:** A comparative pharmacokinetic exposure study was conducted in 24 healthy human subjects. Twelve subjects received remdesivir sublingual tablets and 12 subjects received an intravenous infusion of remdesivir (JUBI-R™) as reference.
The study was a randomized, open-label, balanced, two-treatment, one-sequence, one-period, multiple-dose, parallel design, comparative pharmacokinetic exposure evaluation study of test formulation (remdesivir sublingual tablet 20 mg) with the reference product (remdesivir for injection 100 mg). The study was carried out in healthy adult subjects, under fasting conditions.
Healthy human volunteers who were willing to participate in the study and fulfill the inclusion and exclusion criteria were selected for the study. Volunteers aged from 18-45 years with a bodyweight 50-80 Kg in case of male and/or 45-75 Kg in case of a female with body mass index (BMI) in the range of 18.50-24.90 Kg/m² (both inclusive, calculated as weight in kg/height in meter) were selected for the study. The patients were determined to be healthy as determined by medical history, clinical and laboratory examination performed within 21 days before admission for the study.
**Dosing Procedure:** Sublingual tablet (test): 100 mg twice daily on day 1 followed by 100 mg twice daily from day 2 through day 5. IV infusion (reference): 200 mg (loading dose) on day 1 followed by 100 mg once daily from day 2 through day 5.
**Sampling Schedule:** The blood sample was collected from day 1 to day 4 pre-dose (0.00 h) and day 5: up to 24.0 hr post-dose.
**PK Parameters:** PK parameters were Cₘₐₓ, AUC₀₋ₜₐᵤ, Cₜₐᵤ, and Cₘᵢₙ for nucleoside metabolite (GS-441524).
**Evaluation Criteria:** Ratio of geometric least-square means of the test (T) and reference (R) formulations calculated and reported in percentage for Ln-transformed Cₘₐₓ, Cₜₐᵤ, Cₘᵢₙ, and AUC₀₋ₜₐᵤ for the predominantly circulating nucleoside metabolite (GS-441524).
**Pharmacokinetic Analysis:** Remdesivir sublingual tablets administered to the healthy humans were well tolerated by all the subjects and no safety/tolerability concerns were identified during this study.

**Table 17: Pharmacokinetic Parameters of Example 15 in Humans**

| **Parameter** | **Cₘₐₓ [ng/mL]** | **Cₜₐᵤ [ng/mL]** | **Cₘᵢₙ [ng/mL]** | **AUC₀₋ₜₐᵤ [ng*hr/mL]** |
|---|---|---|---|---|
| **Geometric Mean (T)** | 197.4 | 59.1 | 49.4 | 2373 |
| **Geometric Mean (R)** | 191.5 | 78.8 | 76.4 | 2667.5 |
| **Point Estimate (T/R Ratio) (%)** | **103.1** | **74.9** | **64.7** | **88.9** |

**Conclusion:** These findings confirm that the drug was able to undergo absorption when administered by the sublingual route in human subjects. It rapidly converted to its metabolite form (GS-441524). Good exposure to metabolite (GS-441524) was obtained. Remdesivir sublingual tablets were well tolerated by all the subjects with no safety/tolerability concerns being identified.
There were no serious adverse events reported during any of the studies.
Further analysis was performed with 96 subjects (48 subjects each for test and reference) concentration data generated following bootstrapping statistical procedure for estimating power and confidence intervals. In bootstrapping analysis more than 90% power was achieved for all the primary pharmacokinetic parameters.

## Claims

1. A transmucosal pharmaceutical composition in a solid dosage form comprising an antiviral drug in an amount of about 0.1 mg to about 100 mg and one or more pharmaceutically acceptable excipients, wherein the composition has a pH from about 2.5 to about 7.0 and the composition releases at least 75% of the drug in 15 minutes in 900 ml of McIlvaine Buffer (pH 3.0), using a USP I apparatus (basket) at a temperature of 37±0.5°C and a rotation speed of 100 revolutions per minute.

2. The pharmaceutical composition according to claim 1, wherein the antiviral drug is selected from the group consisting of remdesivir, favipiravir, baloxavir marboxil, besifovir, raltegravir, molnupiravir, GS-441524, and ravidasvir, or any combinations thereof.

3. The pharmaceutical composition according to claim 1, wherein one or more pharmaceutically acceptable excipients are selected from the group comprising diluent, binder, disintegrant, solubilizing agent, surfactant, pH regulating agent, sweetener, taste masking agent, flavoring agent, glidant, and lubricant.

4. The pharmaceutical composition according to claim 3, wherein the diluent comprises one or more of mannitol, lactose, microcrystalline cellulose, silicified microcrystalline cellulose, sucrose, starch, pregelatinized starch, xylitol, sorbitol, maltodextrin, polydextrose, isomalt, and mixtures thereof.

5. The pharmaceutical composition according to claim 3, wherein the solubilizing agent comprises one or more of cyclodextrin, sodium lauryl sulfate, sodium carboxymethylcellulose, povidone, or mixtures thereof.

6. The pharmaceutical composition according to claim 3, wherein the sweetener comprises one or more sucrose, sucralose, glucose, dextrose, saccharin sodium, aspartame, acesulfame potassium, neohesperidine dihydrochalcone, mannitol, xylitol, and thaumatin, and mixtures thereof.

7. The pharmaceutical composition according to claim 3, wherein the flavoring agent comprises one or more menthol, orange, grape, cherry, bubble gum flavor, tutti-frutti flavor, peppermint flavor, bitter taste masker flavor, and mixtures thereof.

8. The pharmaceutical composition according to claim 3, wherein the pH regulating agent comprises one or more of organic acids, inorganic acids, organic bases, inorganic bases amino acids, and mixtures thereof.

9. The pharmaceutical composition according to claim 1, wherein the composition is stable for at least one month while stored at 40°±2°C and 75±5% relative humidity.

10. The pharmaceutical composition according to claim 1, wherein the composition comprises:
a) from about 0.1% to about 35% by weight of an antiviral drug,
b) from about 20% to about 70% by weight of one or more diluents,
c) from about 0.1% to about 10% by weight of one or more disintegrants,
d) from about 0.01% to about 35% by weight of one or more solubilizing agents,
e) from about 0.01% to about 10% by weight of one or more sweeteners,
f) from about 0.01% to about 5% by weight of one or more taste masking agents,
g) from about 0.01% to about 5% by weight of one or more flavoring agents, and
h) one or more pH regulating agents.

11. The pharmaceutical composition according to claim 10, further comprising from about 0.01% to about 3% by weight of one or more glidants and from about 0.01% to about 3% by weight of one or more lubricants.

12. A pharmaceutical composition in the form of a tablet for sublingual administration comprising:
a) an antiviral drug selected from the group comprising remdesivir, favipiravir, baloxavir marboxil, besifovir, raltegravir, molnupiravir, GS-441524, and ravidasvir in an amount from about 0.1 mg to about 100 mg,
b) a pH regulating agent capable of maintaining the pH from about 2.5 to about 7.0,
c) a solubilizing agent, present in an amount such that the ratio of the weight of antiviral drug to the weight of the solubilizing agent is from 1:10 to 10:1,
d) and one or more pharmaceutically acceptable excipients,
wherein the composition releases at least 75% of the drug in 15 minutes in 900 ml of McIlvaine Buffer (pH 3.0), using a USP I apparatus (basket) at a temperature of 37±0.5°C and a rotation speed of 100 revolutions per minute.

13. A sublingual tablet pharmaceutical composition comprising remdesivir or its pharmaceutically acceptable salts or solvates thereof in an amount of about 0.1 mg to about 50 mg, and one or more pharmaceutically acceptable excipients, wherein said sublingual composition provides a pharmacokinetic profile substantially equivalent to the pharmacokinetic profile of intravenous injectable dosage form comprising remdesivir.

14. The pharmaceutical composition according to claim 13, wherein the composition is for at least 20 mg to 200 mg per day administration for a treatment duration of at least three days and wherein said composition, when administered to a human subject, provides a value of AUC₀₋ₜ for GS-441524 test to reference least squares mean ratio where the 90% confidence interval is between 80% and 125% of the natural-log transformed AUC₀₋ₜ value obtained with said commercially available remdesivir injection, and a value of AUC_{0-inf} GS-441524 test to reference least squares mean ratio where the 90% confidence interval is between 80% and 125% of the natural-log transformed AUC_{0-inf} value obtained with said commercially available remdesivir injection.

15. The pharmaceutical composition according to claim 13, wherein the composition exhibits a Cₘₐₓ of about 162.0±55.4 ng/mL for GS-441524, following administration of the sublingual composition to an adult human under fasted conditions.
